# EUROPEAN PATENT SPECIFICATION

(11) **EP 4 197 573 B1**
(45) Date of publication and mention of the grant of the patent: **28.10.2026**
(21) Application number: 21855983.9
(22) Date of filing: 11.08.2021
(51) Int. Cl.: A61M 5/158, A61M 25/06

(54) **WINGED NEEDLE ASSEMBLY, PACKAGING MATERIAL FOR WINGED NEEDLE ASSEMBLY, CAP FOR WINGED NEEDLE ASSEMBLY, AND WINGED NEEDLE ASSEMBLY SET**
NADELANORDNUNG MIT FLÜGELN, VERPACKUNGSMATERIAL FÜR NADELANORDNUNG MIT FLÜGELN, KAPPE FÜR NADELANORDNUNG MIT FLÜGELN UND NADELANORDNUNGSSATZ MIT FLÜGELN
ENSEMBLE AIGUILLE À AILETTES, MATÉRIAU DE CONDITIONNEMENT POUR ENSEMBLE AIGUILLE À AILETTES, CAPUCHON POUR ENSEMBLE AIGUILLE À AILETTES, ET JEU D'ENSEMBLE AIGUILLE À AILETTES

(30) Priority: 14.08.2020 JP 2020136979; 30.09.2020 JP 2020165184
(43) Date of publication of application: 21.06.2023
(73) Proprietor: Nipro Corporation, Osaka-shi, Osaka 531-8510 (JP)
(72) Inventor: NAKAGAMI, Hiroyuki, Osaka-shi, Osaka 531-8510 (JP); TSUCHITORI, Yasuki, Osaka-shi, Osaka 531-8510 (JP); MIYAKE, Takako, Osaka-shi, Osaka 531-8510 (JP); MASUTANI, Akiko, Osaka-shi, Osaka 531-8510 (JP); NAKAGAWA, Naomi, Osaka-shi, Osaka 531-8510 (JP); KUDO, Tatsuya, Osaka-shi, Osaka 531-8510 (JP)
(74) Representative: Wunderlich & Heim Patentanwälte PartG mbB
(86) International application number: PCT/JP2021/029672
(87) International publication number: WO 2022/034904

(56) References cited:
- EP-A1- 1 306 097
- WO-A1-2014/033901
- WO-A1-2017/033449
- WO-A1-2017/033449
- WO-A1-2019/053568
- JP-A- 2000 084 076
- JP-A- S4 870 395
- JP-U- 3 201 367
- US-B2- 7 153 293

## Description

### TECHNICAL FIELD

The present invention relates to a winged needle assembly, and more particularly to a winged needle assembly including an erroneous puncture-preventing mechanism that accommodates a needle tip in a housing using a spring.

### BACKGROUND

Conventionally, a winged needle assembly having wings is used when injecting a drug solution or the like into a patient, or performing treatment such as artificial dialysis or blood sampling. The wings are generally made of a flexible resin, and are used to fix an assembly to an arm or the like of a patient using tape after puncturing with a needle, and are also used for performing puncturing. As a winged needle assembly, those having various structures are known, and for example, a winged needle assembly having an erroneous puncture-preventing mechanism has also been developed. JP 2018-89070 A discloses a winged needle assembly including an erroneous puncture-preventing mechanism in which a needle tip is accommodated in a housing by sliding a needle hub to which a needle is fixed toward a base end. A mechanism for locking the slide of the needle hub is also provided so that the needle accommodated in the housing does not protrude again.

The winged needle assembly of JP 2018-89070 A includes a needle hub in which a locking part is formed, a tubular housing that slidably supports the needle hub, a spring that biases the needle hub toward a base end, and wings provided at a tip end portion of the housing. The housing includes an opening into which the locking part of the needle hub is fitted, and is provided with a flexible piece including a pressing protrusion arranged to face the opening. In this winged needle assembly, the locking part of the needle hub is pushed inward to be detached from the opening of the housing by pressing the flexible piece after use. At this time, the needle hub slides toward the base end due to a biasing force of the spring, and the needle tip is accommodated in the housing.

EP 1 306 097 A1, WO 2019/053568 A1, WO 2017/033449 A1, US 7 153 293 X6, WO 2014/033 901 A1 and JP 2000-084 076 A disclose various variants of needle assemblies for fluid collection with retractable needle and other safety measures including caps.

### SUMMARY

### TECHNICAL PROBLEM

Meanwhile, in a case where a blood vessel of a patient is punctured with a needle in a winged needle assembly, the puncture is generally performed with the wings gripped, but when the wings are gripped, a flexible piece of a housing may be erroneously pressed to accommodate the needle tip in the housing, which locks a needle hub. Therefore, it is required to prevent such erroneous operation. On the other hand, when the flexible piece is intentionally operated, it is necessary to ensure good operability similar to the conventional configuration.

Another object of the winged needle assembly is to realize a stable and rapid puncture operation. Since the operating winged needle assembly is required to be small, it is difficult for some users to grip the wings and they may not be able to quickly shift to the puncture operation.

An object of the present invention is to solve at least one of the above problems.

### SOLUTION TO PROBLEM

A winged needle assembly according to the present invention is a winged needle assembly with the features of claim 1 provided with an erroneous puncture-preventing mechanism for a needle, the winged needle assembly inter alia including: a needle hub to which a base end portion of the needle is fixed and in which a locking part is formed; a housing with a cylindrical shape that supports the needle hub at a first position where the needle protrudes from a cylinder tip; a spring that biases the needle hub toward a base end of the housing; a wing provided in the housing; and an erroneous operation-inhibiting part that suppresses an erroneous operation of the erroneous puncture-preventing mechanism, wherein the housing includes a locked part to which the locking part is locked at a first position of the needle hub and an operation portion that presses the locking part, and the erroneous operation-inhibiting part is a wall portion located between the wing and the locked part.

As described above, the wing provided in the housing is gripped at the time of the puncture operation, but at this time, the operation portion may be erroneously pressed to accommodate the needle tip in the housing, which may lock the needle hub. With the above configuration, it is possible to suppress the erroneous operation of the erroneous puncture-preventing mechanism by the erroneous operation-inhibiting part. On the other hand, the intentional pressing operation of the operation portion can be easily performed by avoiding the erroneous operation-inhibiting part.

In the winged needle assembly according to the present invention, the erroneous operation-inhibiting part is a protruding wall that is formed between the locked part and the wing on the outer peripheral surface of the housing and that protrudes beyond a tip end of the locking part. Since the protruding wall protrudes beyond the tip end position of the locking part, the protruding amount is large, and at the time of the puncture operation in which the wing is gripped, for example, the protruding wall serves as a barrier to effectively suppress an unintended pressing operation of the operation portion.

In the winged needle assembly according to one preferred aspect of the present invention, the wing includes a pair of wing portions extending in a direction orthogonal to an axial direction of the housing and a cylindrical portion to which the pair of wing portions are connected and that is externally fitted to the housing, and the erroneous operation-inhibiting part is provided in the cylindrical portion of the wing and protrudes to a position overlapping at least a tip end of the locking part in the axial direction of the housing. Also in this case, at the time of the puncture operation in which the wing is gripped, the erroneous operation-inhibiting part provided in the cylindrical portion of the wing becomes an obstacle, and an unintended pressing operation of the operation portion is effectively suppressed.

### ADVANTAGEOUS EFFECTS OF INVENTION

According to the present invention, in a winged needle assembly including an erroneous puncture-preventing mechanism that accommodates a needle tip in a housing using a spring, it is possible to suppress an erroneous operation of the erroneous puncture-preventing mechanism while securing favorable operability. Further, according to one aspect of the present invention, stable and rapid operation is possible in the puncture operation using the winged needle assembly.

### BRIEF DESCRIPTION OF DRAWINGS

Fig. 1 is a perspective view of a winged needle assembly of a first embodiment.
Fig. 2 is an exploded perspective view of the winged needle assembly of the first embodiment.
Fig. 3 is a cross-sectional view of the winged needle assembly of the first embodiment.
Fig. 4 is a perspective view of the winged needle assembly of the first embodiment, illustrating a state in which a needle tip is accommodated in a cylinder of a housing.
Fig. 5 is a cross-sectional view of the state illustrated in Fig. 4.
Fig. 6 is a view illustrating a state of puncture operation using the winged needle assembly of the first embodiment.
Fig. 7 is a perspective view of a winged needle assembly of a first example serving to explain aspects of the invention.
Fig. 8 is a view for explaining a configuration of an erroneous operation-inhibiting part in the winged needle assembly of the first example.
Fig. 9 is a view illustrating a modification of the first example.
Fig. 10 is a perspective view of a winged needle assembly of a second example serving to explain aspects of the invention.
Fig. 11 is a perspective view of a stopper constituting the winged needle assembly of the second example.
Fig. 12 is a view illustrating a modification of the second example.
Fig. 13 is a perspective view of a winged needle assembly set of a first example serving to explain aspects of the invention.
Fig. 14 is an exploded perspective view of the winged needle assembly set of the first example.
Fig. 15 is a cross-sectional view of the winged needle assembly set of the first example.
Fig. 16 is a perspective view of the winged needle assembly of another example serving to explain aspects of the invention.
Fig. 17 is a perspective view of a packaging case for the winged needle assembly of the first embodiment.
Fig. 18 is a view illustrating a state of puncture operation using the winged needle assembly of the first embodiment.
Fig. 19 is a perspective view of a winged needle assembly set of the second example serving to explain aspects of the invention.
Fig. 20 is a cross-sectional view of the winged needle assembly set of the second example.
Fig. 21 is a plan view illustrating a modification of the winged needle assembly.

### DESCRIPTION OF EMBODIMENTS

Hereinafter, an embodiment of a winged needle assembly according to the present invention and further unclaimed examples serving to explain aspects of the invention will be described in detail with reference to the drawings. The embodiment and examples described below are merely examplary, and the present invention is not limited to the following embodiment. In addition, it is assumed from the beginning that components of a plurality of embodiments, examples and modifications described below are selectively combined.

### [Winged Needle Assembly]

### <First Embodiment>

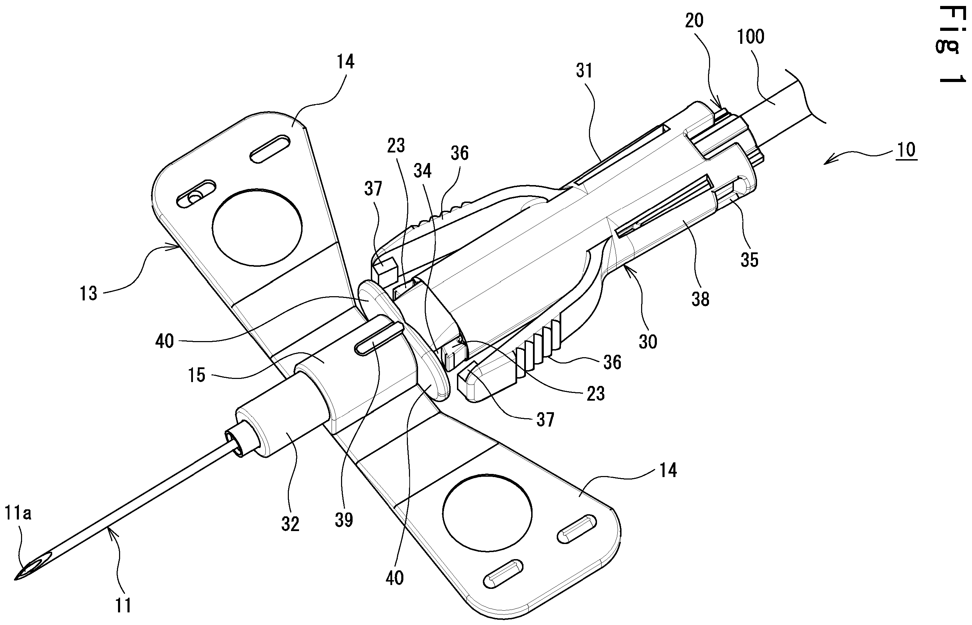

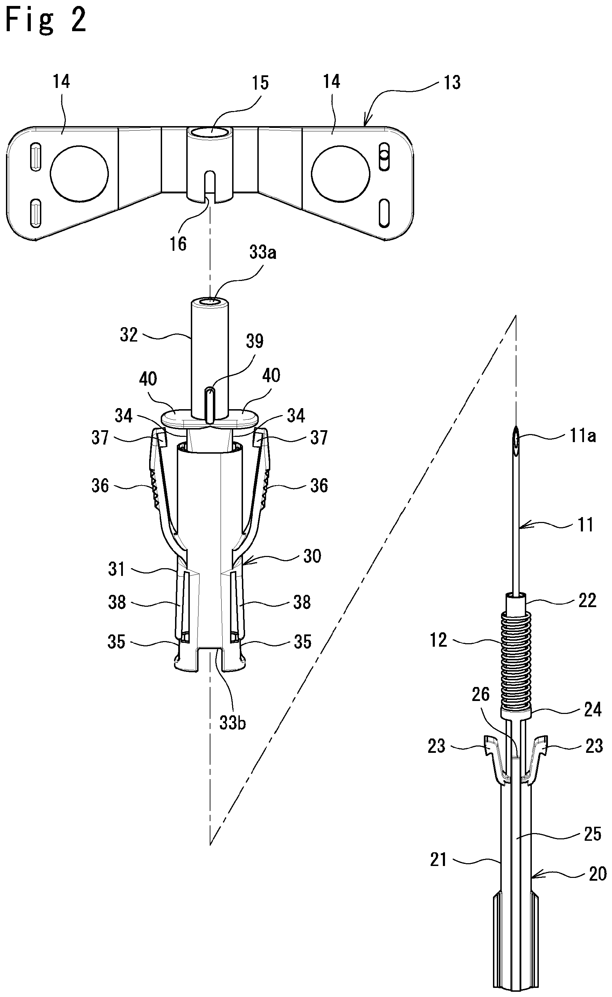

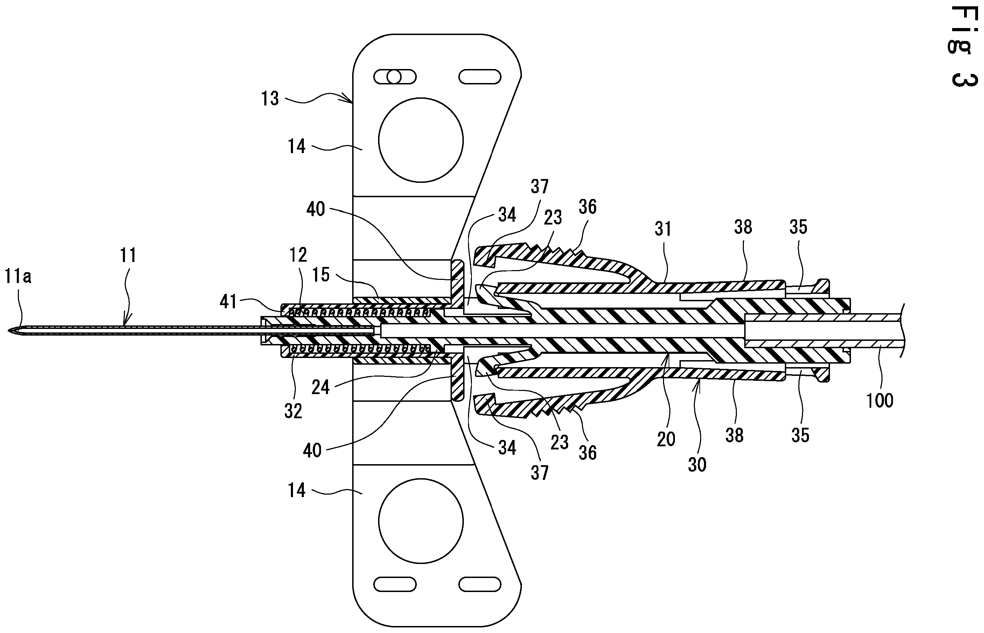

A winged needle assembly 10 according to a first embodiment will be described in detail with reference to Figs. 1 to 6. Fig. 1 is a perspective view of a winged needle assembly 10, and Fig. 2 is an exploded perspective view of the winged needle assembly 10. Fig. 3 is a cross-sectional view of the winged needle assembly 10 taken along the axial direction of a housing 30 at a portion where a flexible piece 36 is formed.

As illustrated in Figs. 1 to 3, the winged needle assembly 10 includes a needle 11, a needle hub 20 to which a base end portion of the needle 11 is fixed, and a housing 30 with a cylindrical shape that accommodates the needle hub 20. The housing 30 supports the needle hub 20 at a first position where the needle 11 protrudes from a cylinder tip. In addition, the housing 30 supports the needle hub 20 in a slidable state from the first position to a second position where the needle 11 is accommodated in the cylinder. The winged needle assembly 10 has a structure where the needle hub 20, into which the needle 11 is fixed, is inserted into the housing 30 that has a cylindrical shape, so as to be movable in the axial direction. In addition, the winged needle assembly 10 includes a spring 12 that biases the needle hub 20 toward the base end of the housing 30 and a wing 13 provided to the housing 30.

In the present embodiment, a tube 100 is connected to the base end portion of the needle hub 20. In the present description, the tip end side of the winged needle assembly 10 means a needle tip 11a side of the needle 11, and the base end side of the winged needle assembly 10 means the side opposite to the needle tip 11a and to which a pipe such as the tube 100 is connected. In the following description, for convenience of explanation, a direction in which wing portions 14 of the wing 13 extend is referred to as a "left-right direction", and a direction orthogonal to the left-right direction and the length direction (axial direction) of the needle 11 is referred to as a "vertical direction".

The winged needle assembly 10 includes an erroneous puncture-preventing mechanism that accommodates the needle 11 in the housing 30 using the spring 12. As a component of the erroneous puncture-preventing mechanism, a locking part 23 is formed in the needle hub 20. The housing 30 has, as locked parts to which the locking part 23 of the needle hub 20 is locked, a first opening 34 to which the locking part 23 is locked at the first position of the needle hub 20 where the needle 11 protrudes from the tip end of the housing 30, and a second opening 35 to which the locking part 23 is locked at the second position of the needle hub 20 where the needle 11 is accommodated in the housing 30. In addition, a flexible piece 36 is provided as operation portions for pressing the locking part 23. In the housing 30, the spring 12 that biases the needle hub 20 toward the base end is arranged.

The winged needle assembly 10 is used in a state in which the locking part 23 of the needle hub 20 is fitted into the first opening 34 of the housing 30 and the needle 11 protrudes from the tip end of the housing 30. After use, the locking part 23 is fitted into the second opening 35, and the needle 11 is accommodated in the housing 30. As will be described in detail later, when the flexible piece 36 is operated to be pressed after use, the locking part 23 fitted to the first opening 34 is pressed toward the inside of the housing 30 by a pressing protrusion 37 of the flexible piece 36 and is detached from the first opening 34. At this time, the needle hub 20 slides toward the base end due to a biasing force of the spring 12, the needle 11 is accommodated in the housing 30, and also the locking part 23 is fitted into the second opening 35 to lock the slide of the needle hub 20.

Hereinafter, each component of the winged needle assembly 10 will be described in detail particularly regarding the configuration of the housing 30.

### [Needle Hub, Needle, Spring]

As illustrated in Figs. 1 to 3, the needle 11 is, for example, a hollow needle made of a metal such as stainless steel, and has the needle tip 11a that is sufficiently sharp to easily puncture the skin, and is fixed to the tip end portion of the needle hub 20. The base end portion of the needle 11 is inserted into the needle hub 20 from the tip end of the needle hub 20, and the hollow portion of the needle 11 communicates with an internal flow path of the needle hub 20. The length of the needle 11 is shorter than the axial length of the housing 30, and the entire needle 11 is accommodated in the cylinder of the housing 30 after use.

The needle hub 20 is a cylindrical member to which the needle 11 is fixed, and is accommodated in the cylinder of the housing 30 in a state of being biased toward the base end of the housing 30 by the spring 12. The needle hub 20 is formed in a substantially cylindrical shape as a whole and has such a diameter that it can be inserted into the cylinder of the housing 30. The needle hub 20 is made of, for example, a hard resin. In the cylinder of the needle hub 20, a flow path through which a medicinal solution or blood passes is formed, and as described above, this flow path communicates with the hollow portion of the needle 11. The flow path in the needle hub 20 is narrow on the tip end side and wide on the base end side, and communicates with the hollow portion of the tube 100.

In the needle hub 20, a large-diameter portion 21 having a large outer diameter is formed on the base end side with respect to the axial central portion, and a small-diameter portion 22 having a small outer diameter is formed on the tip end side. In addition, a pair of locking parts 23 are formed at an axially central portion of the needle hub 20. In the present embodiment, the pair of locking parts 23 extend from the outer peripheral surface at the boundary between the large-diameter portion 21 and the small-diameter portion 22. A flange portion 24 protruding in the radial direction is formed on the tip end side with respect to the locking parts 23 of the needle hub 20. The flange portion 24 is an annular projecting portion formed along the circumferential direction of the needle hub 20 (small-diameter portion 22), and the spring 12 abuts on the flange portion 24. Note that the locking parts 23 may be a separate body from the cylindrical portion of the needle hub 20, or a separate locking part may be attached to the cylindrical portion.

A compression spring is used as the spring 12 that biases the needle hub 20 toward the base end of the housing 30. The spring 12 is, for example, a compression coil spring that is a spirally wound metal wire, and presses the flange portion 24 of the needle hub 20 toward the base end. The spring 12 is externally fitted from the tip end of the needle hub 20 to the small-diameter portion 22, and is arranged in the housing 30 in a state of being compressed by the flange portion 24 and an annular portion 41 (see Fig. 3) formed at the tip end portion of the housing 30.

In the needle hub 20, a projecting strip portion 25 is formed from the flange portion 24 to the base end. The projecting strip portion 25 is a projecting portion formed by causing a part of the outer peripheral surface of the needle hub 20 to protrude in the radial direction, and is formed along the axial direction of the needle hub 20. For example, a pair of the projecting strip portions 25 is formed above and below the needle hub 20. In the projecting strip portion 25, a step 26 is formed at a portion sandwiched between the pair of locking parts 23, and the height of the projecting strip portion 25 is higher on the base end side than the step 26.

Note that a groove (not illustrated) into which a portion of the projecting strip portion 25 on the base end side with respect to the step 26 is fitted is formed in the cylinder of the housing 30. The uneven structure of the groove and the projecting strip portion 25 guides the movement of the needle hub 20 along the axial direction of the housing 30, and the step portion of the projecting strip portion 25 contacts with the tip end portion of the groove, whereby the movement of the needle hub 20 toward the tip end is restricted.

As described above, the locking parts 23 are portions fitted to the opening portions of the housing 30 and extend from the outer peripheral surface of the needle hub 20. One locking part 23 is formed on each of the left and right sides of the needle hub 20, and extends in the direction toward the tip end from the axial central portion of the needle hub 20. In addition, the pair of locking parts 23 are inclined with respect to the axial direction of the needle hub 20 so that the distance between the locking parts gradually increases from the root toward the tip end.

Each tip end portion of the pair of locking parts 23 is bent outward to protrude to the left and right so as to be easily caught by the opening portions of the housing 30. In addition, the locking parts 23 are configured to be elastically deformed in the radial direction (left-right direction) of the needle hub 20. That is, the pair of locking parts 23 are elastically deformable such that the distance between them changes. The locking parts 23 are arranged in the housing 30 in a state, for example, of being slightly compressed radially inward.

### [Housing, Wings]

As illustrated in Figs. 1 to 3, the housing 30 is a cylindrical member that accommodates the needle hub 20, and is formed in a substantially cylindrical shape as a whole. In the present embodiment, the tip end portion of the needle hub 20 protrudes from an opening 33a on the tip end side of the housing 30, and the base end portion of the needle hub 20 protrudes from an opening 33b on the base end side of the housing 30. That is, the length of the housing 30 is slightly smaller than that of the needle hub 20. The housing 30 is made of, for example, a hard resin.

In the housing 30, a cross section of a tip end side portion has a circular shape, and a cross section of a base end side portion has a substantially elliptical shape having a long diameter along the left-right direction. The base end side of the housing 30 is a large-diameter portion 31 having a large outer diameter, and the tip end side is a small-diameter portion 32 having a small outer diameter, and the length of the large-diameter portion 31 is larger than that of the small-diameter portion 32. A pair of first openings 34, a pair of second openings 35, and a pair of flexible pieces 36 are formed on both left and right sides of the large-diameter portion 31. In addition, a protruding wall 40 is formed on the tip end side of the first openings 34 as an erroneous operation-inhibiting part that suppresses erroneous operation of the erroneous puncture-preventing mechanism due to mishandling of the flexible pieces 36.

The wing 13 is attached to the housing 30 on the tip end side of the protruding wall 40. The wing 13 is, for example, a flexible rubber member, and is used when performing a puncture operation, and is also used when fixing the winged needle assembly 10 to an arm or the like of a patient, using tape. The wing 13 includes a pair of wing portions 14 extending in the left-right direction orthogonal to the axial direction of the housing 30, and a cylindrical portion 15 to which the root of each wing portion 14 is connected, and is attached to the housing 30 by the cylindrical portion 15 externally fitted to the small-diameter portion 32 of the housing 30. The wing portions 14 extend largely from a lower end portion of the cylindrical portion 15 to both left and right sides beyond both left and right ends of the flexible pieces 36, and their tip ends are formed to be wider than their roots.

A projecting strip portion 39 extending from the protruding wall 40 toward the tip end is formed at an upper end portion of the small-diameter portion 32 of the housing 30, and a recess portion 16 into which the projecting strip portion 39 is fitted is formed at an upper end portion of the cylindrical portion 15 of the wing 13. With this uneven structure, the wing 13 is positioned in the circumferential direction with respect to the housing 30. In addition, the housing 30 has an annular portion 41 protruding in the center direction of the opening 33a and formed in an annular shape along the peripheral edge of the opening 33a. As described above, the spring 12 is arranged in a compressed state between the flange portion 24 and the annular portion 41 of the needle hub 20. As a result, a biasing force for sliding the needle hub 20 toward the base end of the housing 30 is obtained.

One first opening 34 is formed on each of the left and right sides through the cylinder wall at the tip end portion of the large-diameter portion 31. The first opening 34 is a locked part into which the locking part 23 of the needle hub 20 is fitted in a use state in which the needle 11 protrudes from the tip end of the housing 30 (that is, the first position of the needle hub 20), and is formed in a size into which the tip end portion of the locking part 23 can be inserted. The tip end portion of the locking part 23 is caught by a base end side edge of the first opening 34. The pair of first openings 34 are, for example, formed in a rectangular shape having the same size.

The cylinder wall of the housing 30 has an inner diameter in the left-right direction that is smaller than the interval between the pair of locking parts 23 at a portion adjacent to the base end side of the first opening 34. In this case, since the pair of locking parts 23 is compressed radially inward, the needle hub 20 is stably fixed to the housing 30.

The second opening 35 is formed on each of the left and right sides through the cylinder wall at the base end portion of the large-diameter portion 31. The second opening 35 is a locked part to which the locking part 23 of the needle hub 20 is fitted in a use state in which the needle 11 is accommodated in the housing 30 (that is, the second position of the needle hub 20), and is formed in a size into which the tip end portion of the locking part 23 can be inserted. Similarly to the first openings 34, the pair of second openings 35 are formed in, for example, a rectangular shape having the same size, and the tip end portion of the locking part 23 is caught by a base end side edge of the second opening 35.

An inclined wall portion 38 inclined with respect to the axial direction is formed on the cylinder wall of the housing 30 so as to gradually expand radially outward toward the base end. The inclined wall portion 38 is formed on both left and right sides from the axial central portion of the large-diameter portion 31 to the edge portion of the second opening 35. The edge portion on the tip end side of the second opening 35 is located radially outside the edge portion on the base end side. In this case, when the needle hub 20 slides toward the base end by the biasing force of the spring 12, the locking part 23 is more reliably guided to the second opening 35.

The flexible piece 36 is an operation portion that is operated when the needle 11 is accommodated in the housing 30, and is formed in an arm shape extending from the outer peripheral surface of the large-diameter portion 31. One flexible piece 36 is formed on each of the left and right sides of the housing 30, and extends in the direction toward the tip end from the axial central portion of the large-diameter portion 31. In addition, the pair of flexible pieces 36 are inclined with respect to the axial direction of the housing 30 and extend to a position facing the first openings 34 so that the distance between the flexible pieces gradually increases from the roots toward the tip ends. Note that a configuration in which a spring is provided between the flexible pieces 36 and the outer peripheral surface of the housing 30 to inhibit the displacement or the pressing operation of the operation portion may be provided, but in this case, if the configuration becomes complicated or the spring constant is increased in order to enhance the suppression function, there is a disadvantage in that a pressing force required for the operation increases.

The pair of flexible pieces 36 are configured to be elastically deformed in the radial direction (left-right direction) of the housing 30. Pressing protrusions 37 protruding in the direction of the first openings 34 are formed at the tip end portion of each flexible piece 36. The pressing protrusions 37 are oppositely arranged to face the outside of the first openings 34 at a predetermined distance in the radial direction of the housing 30, and press the locking parts 23 of the needle hub 20 fitted to the first openings 34 when the flexible pieces 36 are pressed. Note that the operation portion that presses the locking part 23 is not limited to the flexible piece 36, and for example, a member separate from the cylindrical portion of the housing 30 may be rotatably attached via a pin or the like to serve as an operation portion.

As described above, the protruding wall 40 is formed in the housing 30 as an erroneous operation-inhibiting part that suppresses erroneous operation of the erroneous puncture-preventing mechanism due to mishandling of the flexible piece 36. The protruding wall 40 is, in the axial direction of the housing 30, a wall portion located between the wing 13 and the flexible piece 36, in other words, between the wing 13 and the first opening 34, and protrudes in a direction intersecting the axial direction of the housing 30. The protruding wall 40 is formed in a plate shape, and its thickness is made thin within a range in which deformation or breakage does not occur when being pressed at the time of puncture operation or the like, from the viewpoint of, for example, downsizing of an instrument or the like.

The protruding wall 40 protrudes in the left-right direction in which the pair of flexible pieces 36 are arranged, at a position close to the tip ends of the flexible pieces 36 and not overlapping the flexible pieces 36 in the left-right direction. By providing this protruding wall 40, the protruding wall 40 serves as a barrier at the time of puncture operation, which suppresses unintended pressing operation of the flexible pieces 36. On the other hand, since the protruding wall 40 does not overlap the flexible pieces 36 in the left-right direction, it does not become an obstacle when the flexible pieces 36 are intentionally pressed, and good operability of the flexible pieces 36 is secured, similarly to the conventional configuration.

The protruding wall 40 is formed between the wing 13 and the first opening 34 on the outer peripheral surface of the housing 30 and protrudes beyond the tip end of the locking part 23 fitted to the first opening 34. The protruding wall 40 is formed adjacent to and separating the wing 13 and the first opening 34. In addition, one protruding wall 40 is formed on each of the left and right sides of the housing 30 to have the same length. The pair of protruding walls 40 extend in parallel along the left-right direction (direction in which the pair of flexible pieces 36 are arranged).

Note that the protruding wall 40 may extend obliquely in the left-right direction so as to gradually approach the flexible piece 36 toward the tip end of the protruding wall 40 within a range not interfering with the flexible piece 36. Alternatively, the protruding wall 40 may extend obliquely in the left-right direction so as to gradually separate from the flexible piece 36 toward the tip end of the protruding wall 40 within a range in which erroneous operation of the flexible piece 36 can be suppressed. Further, the protruding wall 40 may be separate from the cylindrical portion of the housing 30, or a separate protruding wall may be attached to the cylindrical portion.

The protruding wall 40 overlaps the locking part 23 in the axial direction of the housing 30 (see Fig. 3). In the present embodiment, the protruding wall 40 further protrudes to the left and right so that the tip end of the pressing protrusion 37 of the flexible piece 36 and the tip end of the protruding wall 40 overlap in the axial direction of the housing 30. The length along the left-right direction from the tip end of one protruding wall 40 to the tip end of the other protruding wall 40 is larger than the long diameter of the large-diameter portion 31. Note that the pair of protruding walls 40 may protrude to the left and right beyond the left and right ends of the flexible piece 36, but from the viewpoint of downsizing of the instrument, operability at the time of puncture, and the like, it is preferable that the protruding walls are formed to have a length exceeding the tip end positions of the locking parts 23 and not exceeding the left and right ends of the flexible pieces 36.

The protruding wall 40 protrudes only in the left-right direction from the outer peripheral surface of the housing 30, and does not substantially protrude in the vertical direction. The vertical length of the protruding wall 40 is, for example, the same as the vertical length of the large-diameter portion 31. In particular, since the protruding wall 40 does not protrude downward beyond the outer peripheral surface of the large-diameter portion 31, it does not become an obstacle when fixing the instrument to an arm of a patient. In addition, a tip end edge of the protruding wall 40 preferably has a rounded curved shape without corners since the lower portion may contact with the skin of the patient and the upper portion may contact with a fingertip of a user who grips the winged needle assembly 10. The shape of the protruding wall is not limited to a thin plate shape. For example, the protruding wall may have a fan shape in which the lateral dimension decreases toward the needle tip. In the portion of the housing on the tip end side with respect to the protruding wall and the portion of the housing where the protruding wall is located, it is preferable that the portion of the housing where the protruding wall is located has a larger horizontal width and a larger ratio of the width to the height.

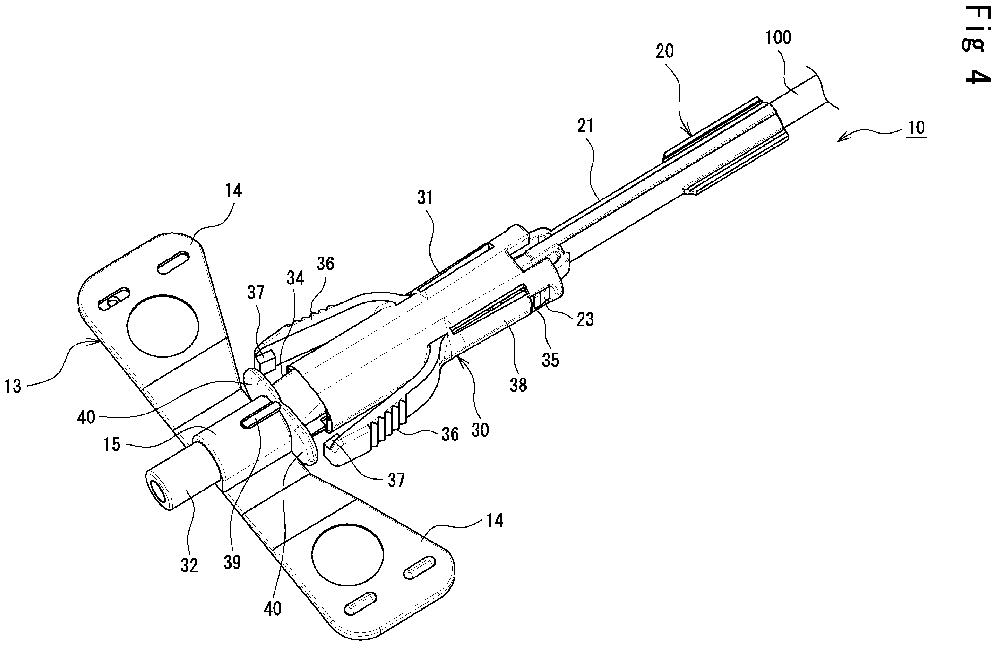

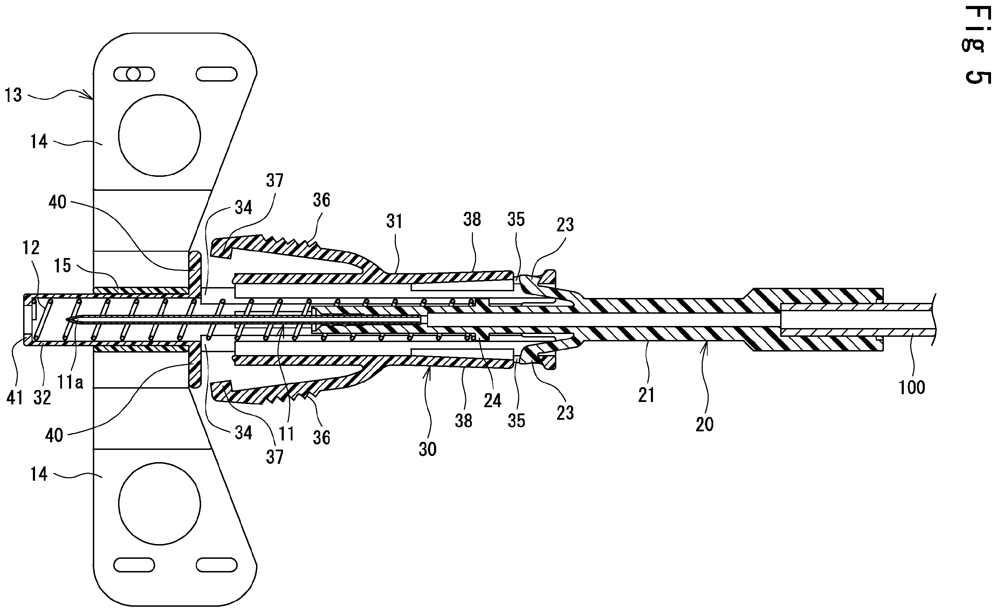

Figs. 4 and 5 are views illustrating a state in which the needle tip 11a is accommodated in the cylinder of the housing 30. As illustrated in Figs. 4 and 5, with the winged needle assembly 10 having the above-described configuration, the needle 11 can be accommodated in the housing 30 after use, and erroneous puncture by the needle 11 can be prevented. When the pair of flexible pieces 36 are pressed in a state in which the needle 11 punctures a blood vessel of a patient after treatment such as infusion or blood sampling is completed, the pressing protrusions 37 of the flexible pieces 36 press the tip end portions of the locking parts 23 of the needle hub 20, that are fitted into the first openings 34, into the housing 30, and the locking parts 23 are elastically deformed to cause the tip end portions of the locking parts 23 to be detached from the first openings 34.

Since the needle hub 20 is biased toward the base end of the housing 30 by the spring 12, when the locking part 23 is detached from the first opening 34, the needle hub 20 slides toward the base end from the first position, and the needle 11 fixed to the tip end portion of the needle hub 20 also slides toward the base end to be accommodated in the housing 30. Then, the locking part 23 is restored and deformed in the process of moving toward the base end of the housing 30, and the tip end portion of the locking part 23 enters the second opening 35 and is caught by the base end side edge of the second opening 35. By fitting the tip end portion of the locking part 23 into the second opening 35, the slide is locked so that the needle hub 20 does not move from the second position, which prevents the re-protrusion of the needle 11. As a result, the winged needle assembly 10 can be safely discarded.

When the needle 11 is accommodated in the housing 30 through the pressing operation of the pair of flexible pieces 36, the central portion in the length direction of each flexible piece 36 is generally pressed from the right and left, and thus the protruding wall 40 formed on the tip end side of the housing 30 with respect to the flexible piece 36 does not interfere with this operation. That is, in the winged needle assembly 10, good operability of the flexible piece 36 is secured, as with the conventional configuration.

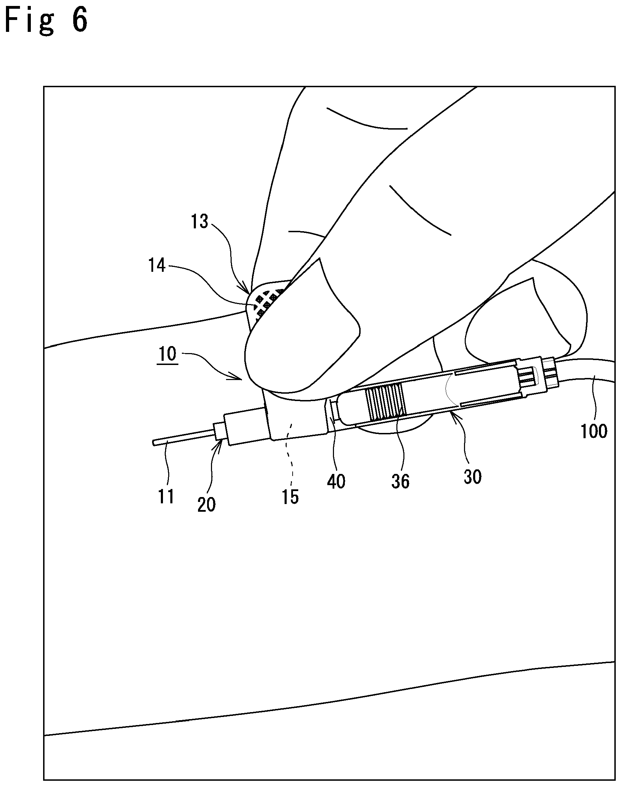

Fig. 6 is a view illustrating a state of puncture operation using the winged needle assembly 10 having the above configuration. As illustrated in Fig. 6, when a blood vessel of a patient is punctured with the needle 11, the wing portions 14 are gripped with the pair of wing portions 14 folded upward and overlapped. That is, the pair of wing portions 14 are gripped while being folded upward to be overlapped so as to sandwich the cylindrical portion 15 of the wing 13, and a blood vessel of a patient is punctured with the needle 11.

At this time, in particular, in a case where the roots of the wing portions 14 are gripped, it is conceivable that a finger may be caught on the flexible piece 36, which would press the flexible piece 36 erroneously, but with the winged needle assembly 10, since the protruding wall 40 greatly protrudes from a position close to the tip end of the flexible piece 36, the protruding wall 40 serves as a barrier to effectively suppress mishandling of the flexible piece 36. Therefore, with the winged needle assembly 10, it is possible to suppress an erroneous operation of the erroneous puncture-preventing mechanism due to mishandling of the flexible piece 36. In particular, in a winged indwelling needle in which a needle tip is accommodated in a housing by a spring, since the winged indwelling needle is required to be small, it is difficult to lengthen the housing in the axial direction, and as a result, to lengthen a portion where the spring is pressed is located on the tip end side of the housing, that is, the side close to the wing. In this case, the flexible piece 36 is likely to be erroneously operated when the wing is gripped. However, since the protruding wall 40 is formed, it is possible to make it difficult to erroneously operate the flexible piece 36 when gripping the wing or the like, which can suppress erroneous operation of the erroneous puncture-preventing mechanism while miniaturization is achieved.

### <First Example>

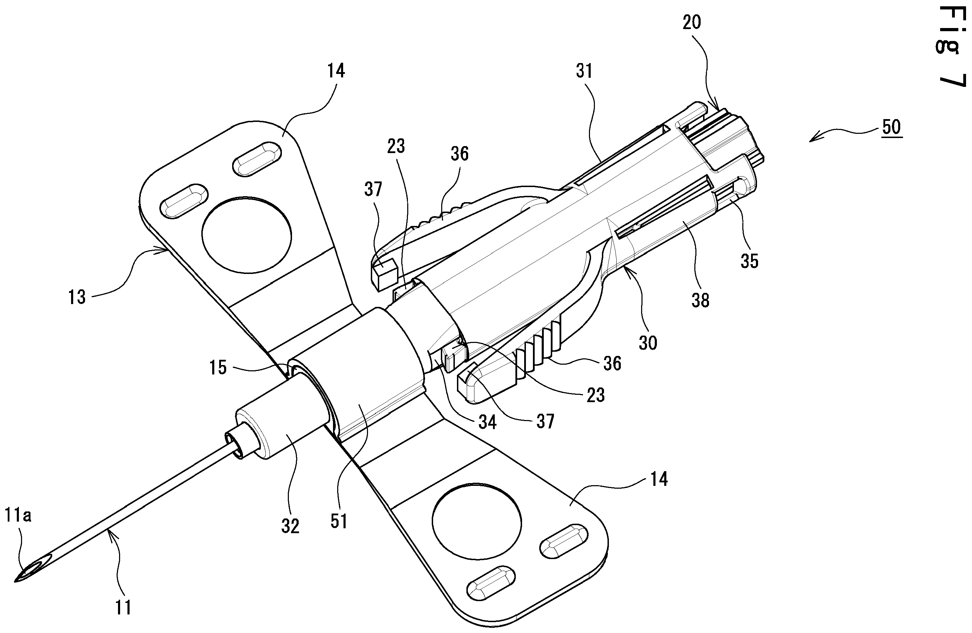

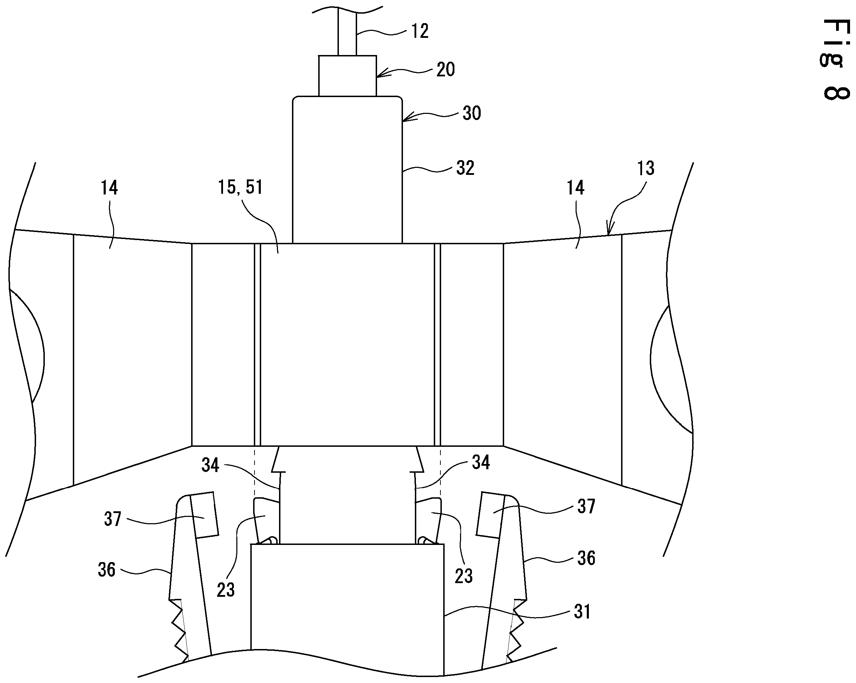

A winged needle assembly 50 according to a first example serving to explain aspects of the invention will be described in detail with reference to Figs. 7 to 9. Fig. 7 is a perspective view of the winged needle assembly 50, and Fig. 8 is a plan view of the winged needle assembly 50 and is an enlarged view of the erroneous operation suppressing member 51 and its vicinity. Hereinafter, the same reference numerals are used for the same or similar components as those in the first embodiment, and redundant description will be omitted.

As illustrated in Figs. 7 and 8, the winged needle assembly 50 includes a needle 11, a needle hub 20, and a housing 30, similarly to the winged needle assembly 10. The winged needle assembly 50 also includes a spring 12 (not shown in Figs. 7 and 8) and a wing 13. On the other hand, the winged needle assembly 50 is different from the winged needle assembly 10 in that no protruding wall 40 exists in the housing 30, and an erroneous operation suppressing member 51 for suppressing erroneous operation of the erroneous puncture-preventing mechanism due to mishandling of the flexible piece 36 is provided in the cylindrical portion 15 of the wing 13.

The erroneous operation suppressing member 51 is an inhibition portion that is provided on the wing 13 and that inhibits bending of the wing portions 14 on the radially inner side of the housing 30 with respect to the locking part 23, and may be detachably attached to the cylindrical portion 15 or may be joined with it using an adhesive or the like. The erroneous operation suppressing member 51 is a semi-cylindrical member that is curved along the outer peripheral surface of the cylindrical portion 15 and that covers the upper portion and both left and right sides of the cylindrical portion 15. As illustrated in Fig. 6, the wing portions 14 are gripped at the time of the puncture operation, and with the winged needle assembly 50, the erroneous operation suppressing member 51 is attached to the cylindrical portion 15 to thicken the root portions of the wing portions 14 to the left and right, so that the thick root portions become an obstacle for suppressing the mishandling of the flexible piece 36.

In the root portion of the wing 13 including the cylindrical portion 15 and the erroneous operation suppressing member 51, that is, a portion surrounding the outer peripheral surface of the small-diameter portion 32 of the housing 30, the left and right side portions are formed thicker than the lower portion. In the present example, since the semi-cylindrical erroneous operation suppressing member 51 having a substantially constant thickness is attached to a portion located above the wing portions 14 of the cylindrical portion 15, the portion other than the lower portion of the root portion of the wing 13 is formed thick. When the thickness of the lower portion forming the lower surface of the wing 13 together with the wing portions 14 in the root portion is increased, the puncturing needle 11 imposes a burden on the blood vessel, and therefore, thickening the left and right side portions without increasing the thickness of the lower portion makes it possible to effectively suppress mishandling of the flexible piece 36 while preventing such a problem.

In a state of being attached to the cylindrical portion 15, the erroneous operation suppressing member 51 preferably protrudes to the left and right up to a position overlapping at least the tip end of the locking part 23 fitted to the first opening 34 in the axial direction of the housing 30 (see Fig. 8). In the present example, the left and right ends of the erroneous operation suppressing member 51 and the tip ends of the pair of locking parts 23 are aligned in the axial direction of the housing 30. Note that at the time of puncture operation, the wing portions 14 are gripped with the pair of wing portions 14 bent upward and overlapped, and therefore, the root portions of the wing portions 14 greatly protrude to the left and right beyond the tip ends of the locking parts 23.

In the winged needle assembly 50, the effect of suppressing mishandling of the flexible piece 36 increases as the thickness of the erroneous operation suppressing member 51 increases, but it is preferable to set the thickness in consideration of the instrument weight and the like. The erroneous operation suppressing member 51 only needs to have a length that does not interfere with the flexible piece 36, and may be formed to have the same length as the cylindrical portion 15. The erroneous operation suppressing member 51 may be provided with a protrusion (wall portion) protruding to the left and right like a stopper 61 to be described later, and the erroneous operation suppressing member 51 may protrude to the left and right to a position overlapping the pressing protrusion 37 in the axial direction of the housing 30.

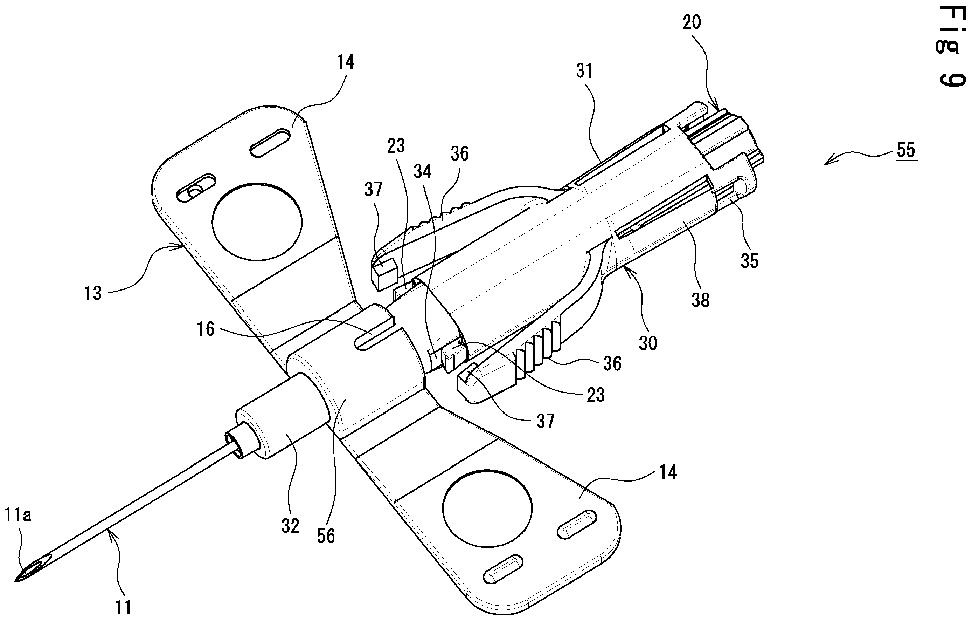

Fig. 9 is a perspective view of a winged needle assembly 55, which is a modification of the winged needle assembly 50. The winged needle assembly 55 is similar to the winged needle assembly 50 in that an erroneous operation-inhibiting part is provided on the wing 13. On the other hand, the winged needle assembly 55 is different from the winged needle assembly 50 in that the cylindrical portion and the erroneous operation-inhibiting part are integrated. That is, the winged needle assembly 55 is provided with an erroneous operation suppression cylindrical portion 56 protruding to the left and right up to a position overlapping at least the tip end of the locking part 23 fitted in the first opening 34 in the axial direction of the housing 30.

The erroneous operation suppression cylindrical portion 56 is provided with a recess portion 16 into which the projecting strip portion 39 of the housing 30 is fitted. The erroneous operation suppression cylindrical portion 56, which is a root portion of the wing 13, has an upper portion and left and right side portions thicker than a lower portion, and an upper end of the cylindrical portion protrudes upward from an outer peripheral surface of the large-diameter portion 31 of the housing 30. In addition, the erroneous operation suppression cylindrical portion 56 may protrude to the left and right from the outer peripheral surface of the large-diameter portion 31. With the winged needle assembly 55, similarly to the winged needle assembly 50, the root portions of the thick wing portions 14 serve as an obstacle, which suppresses mishandling of the flexible piece 36 at the time of puncture operation.

Note that an inhibition portion that inhibits bending of the wing portions 14 on the radially inner side of the housing 30 with respect to the locking part 23 may be provided in the wing portions 14. The inhibition portion is, for example, a plate-shaped member attached to the upper surface of the wing portions 14, and increases the thickness in the vicinity of the roots of the wing portions 14 to increase the rigidity. Note that the thickness may be small as long as the member has high rigidity. In addition, the vicinity of the roots of the wing portions 14 may be formed thick to increase rigidity. Increasing the rigidity in the vicinity of the roots of the wing portions 14 makes it possible to realize a configuration in which the wing is bent with a boundary between a portion having high rigidity and a portion having low rigidity as an inflection point.

### <Second Example>

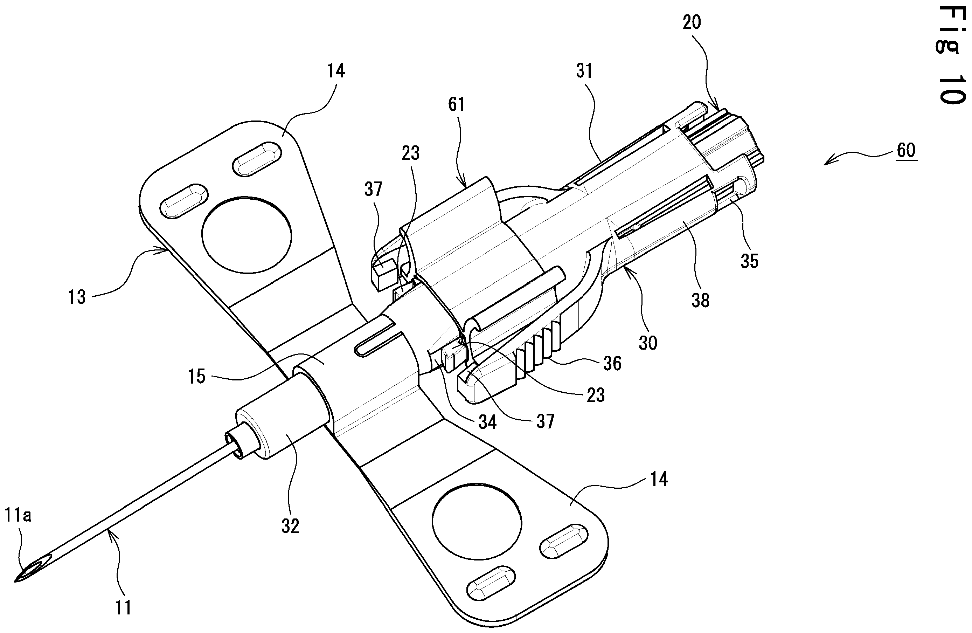

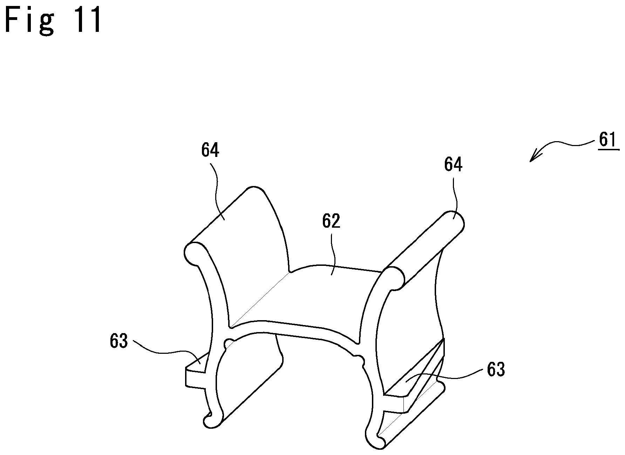

A winged needle assembly 60 according to a second example serving to explain aspects of the invention will be described in detail with reference to Figs. 10 to 12. Fig. 10 is a perspective view of the winged needle assembly 60, and Fig. 11 is a perspective view of the stopper 61. Hereinafter, the same reference numerals are used for the same or similar components as those in the first embodiment and first example, and redundant description will be omitted.

As illustrated in Fig. 10, the winged needle assembly 60 includes a needle 11, a needle hub 20, and a housing 30, similarly to the winged needle assemblies 10 and 50. The winged needle assembly 60 also includes a spring 12 (not shown in Fig. 10) and a wing 13. On the other hand, the winged needle assembly 60 is different from the winged needle assemblies 10 and 50 in including the stopper 61 movably or detachably attached to the outer peripheral surface of the housing 30 as an erroneous operation-inhibiting part.

The stopper 61 is an inhibition portion that inhibits displacement or a pressing operation of the flexible piece 36, which is an operation portion, and is detachably attached to the outer peripheral surface of the housing 30, and is interposed between the outer peripheral surface and the flexible piece 36. That is, the stopper 61 is attached to a portion located inside the flexible piece 36 in the outer peripheral surface of the housing 30. In this case, at the time of the puncture operation, the stopper 61 becomes an obstacle to restrict the movement of the flexible piece 36 toward the inside, which prevents the contact between the pressing protrusion 37 and the locking part 23. Therefore, even if the flexible piece 36 is erroneously pressed, an erroneous operation of the erroneous puncture-preventing mechanism does not occur. Further, by removing the stopper 61, the flexible piece 36 can be pressed as in the conventional configuration.

As illustrated in Figs. 10 and 11, the stopper 61 is curved along the outer peripheral surface of the housing 30, and includes a semi-cylindrical base portion 62 that covers an upper portion and both left and right sides of the housing 30, and a pair of protrusions 63 protruding from the base portion 62 to both left and right sides. The base portion 62 is externally fitted to the large-diameter portion 31 of the housing 30. The protrusion 63 is formed at a position overlapping the flexible piece 36 in the left-right direction. In addition, the protrusion 63 has a substantially triangular shape with a protruding length gradually increasing toward the tip end of the flexible piece 36. In this case, the area of contact with the inner surface of the flexible piece 36 increases, which can suppress unintentional detachment of the stopper 61. The protrusion 63 is formed to such a length that the locking part 23 is not pressed by the pressing protrusion 37 when the flexible piece 36 is pressed.

The stopper 61 has a pair of gripping portions 64 extending upward from both left and right sides of the base portion 62. The gripping portions 64 are formed over the entire length of the base portion 62, and is curved small so that a tip end thereof is positioned slightly outside the root. By pinching the pair of gripping portions 64 from the outside and then pressing them inward, the lower end side of the base portion 62 can be widened, which can easily remove the stopper 61 from the housing 30.

In the present example, the base portion 62 is configured to be elastically deformable in the left-right direction, has an inner diameter slightly smaller than the outer diameter of the large-diameter portion 31, and is attached with the outer peripheral surface of the large-diameter portion 31 pressed. Note that since the stopper 61 is easily deformed, it is not arranged between the locking part 23 and the pressing protrusion 37. The stopper need not include the pair of gripping portions 64, and the gripping portions 64 may be detachable without being pinched from the outside.

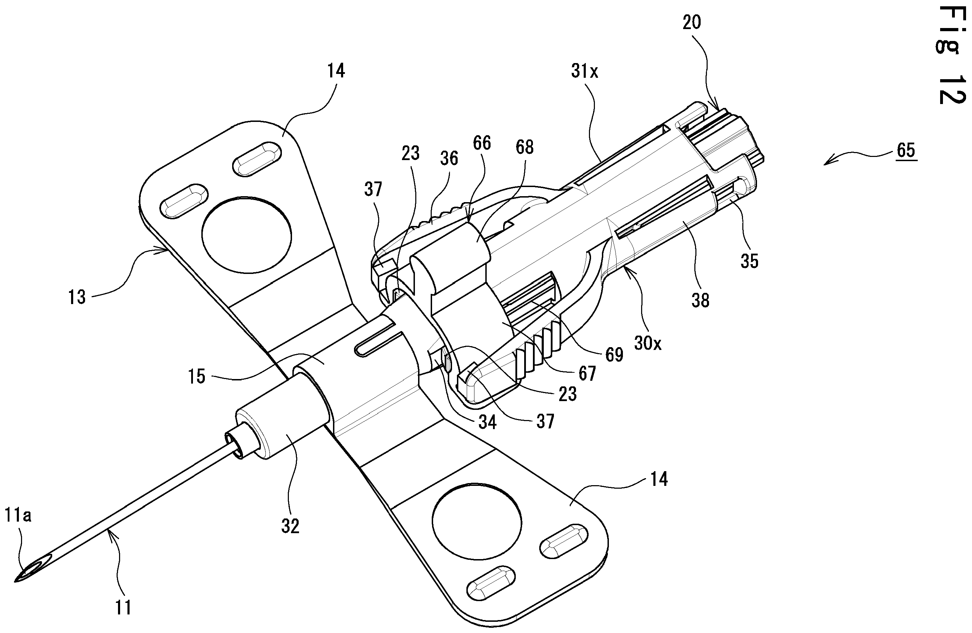

Fig. 12 is a perspective view of a winged needle assembly 65, which is a modification of the winged needle assembly 60. The winged needle assembly 65 is similar to the winged needle assembly 60 in that a stopper 66 attached to the outer peripheral surface of a housing 30x is provided as an erroneous operation-inhibiting part. On the other hand, the winged needle assembly 65 is different from the winged needle assembly 60 in that it is configured so that the stopper 66 is not necessary removed even when the flexible piece 36 is pressed to accommodate the needle 11 in the housing 30x.

Similarly to the stopper 61, the stopper 66 includes a base portion 67 having a semi-cylindrical shape externally fitted to the large-diameter portion 31x of the housing 30x and a gripping portion 68 extending from the base portion 67. The base portion 67 is configured to be elastically deformable in the left-right direction, and has an inner diameter slightly smaller than the outer diameter of the large-diameter portion 31, and is attached with the outer peripheral surface of the large-diameter portion 31 pressed. The gripping portion 68 extends upward from the upper end of the base portion 67. The stopper 66 does not have a protrusion protruding to the left and right, and in the example illustrated in Fig. 12, the stopper 66 is attached to a portion located inside the flexible piece 36 in the outer peripheral surface of the housing 30x so as to cover the locking part 23 fitted to the first opening 34.

A recess portion 69 in which the stopper 66 is accommodated is formed on the outer peripheral surface of the housing 30x. The recess portion 69 is a portion located inside the flexible piece 36 in the outer peripheral surface of the large-diameter portion 31x, and is formed on the base end side of the first opening 34. The recess portion 69 is formed, for example, by cutting out the left and right side portions of the cylinder wall of the large-diameter portion 31x, on each of the left and right sides have a size that allows the base portion 67 of the stopper 66 to be fitted. The recess portion 69 is formed at a depth corresponding to the thickness of the base portion 67.

When fitted in the recess portion 69, the stopper 66 bends inward and its outer diameter decreases. Even in this state, it is preferable that a pressing force acts on the outer peripheral surface of the large-diameter portion 31. In the large-diameter portion 31x, for example, a second recess portion shallower than the recess portion 69 may be formed so that the stopper 66 does not move from the initial attachment position, and a lock mechanism for fixing the stopper 66 after the movement may be provided. In addition, the axial length of the stopper 66 may be shortened so that the tip end side of the flexible piece 36 can be pressed even if the stopper 66 is not bent inward.

With the winged needle assembly 65, when the flexible piece 36 is pressed to accommodate the needle 11 in the housing 30x, moving the stopper 66 to the recess portion 69 enables the pressing operation of the flexible piece 36 without removing the stopper 66 from the housing 30x. This eliminates the necessity to remove the stopper 66 and separately dispose the stopper as waste, which improves the convenience to the user.

Note that the above-described embodiment and examples can be appropriately modified in design without impairing the object of the present invention. For example, the erroneous operation-inhibiting part of the above embodiment is provided on both the left and right sides, but may be provided on only one of the left and right sides.

In the first embodiment, the erroneous operation-inhibiting part may be a wall portion covering a part of the operation portion. As an example, the above wall portion includes a wall portion that protrudes from the large-diameter portion 31 of the housing 30 to the left and right and covers the upper side of the pressing protrusion 37 of the flexible piece 36. The wall portion overlaps a part of the operation portion in the vertical direction, but does not overlap the operation portion in the left-right direction. Therefore, the intentional pressing operation of the operation portion can be easily performed by avoiding the wall portion. Note that the wall portion functioning as an erroneous operation-inhibiting part only needs to be able to alleviate a situation in which the locking part is unlocked from the locked part, and the wall portion need not extend to cover the upper side of the pressing protrusion 37 of the flexible piece 36. In addition, the wall portion is not limited to a wall protruding in the direction perpendicular to the axis of the housing, and may be a wall extending between the operation portion and the locking part along the axial direction, and the wall portion may extend from a member constituting the wing. For example, a wall portion extending in the direction toward the base end from the cylindrical portion may be provided. With the wall portion, even if the operation portion unintentionally contacts the finger when gripping the wing, a configuration can be made so that the finger contacts the wall portion before the locking part is unlocked, and unintentional unlocking of the locking part can be alleviated by attracting the user's attention.

In the first example, the erroneous operation-inhibiting part is formed in the cylindrical portion located at the center of the pair of wings, but may be formed only in the wing portions. In addition, a protruding portion extending radially outward from the outer surface of the cylindrical portion may be provided to restrict the bending of the wing by the protruding portion. For example, it is possible to provide a protruding portion extending in the left-right direction from the top surface of the cylindrical portion, or adopt a shape in which the cylindrical portion has a width in the left-right direction increasing from the top surface toward the bottom surface. In addition, the inflection point of the bending of the wing can be set at a desired position by, for example, forming the wing with different materials by two-color molding or the like, providing a plurality of linear projecting portions, providing a linear rib, or the like.

In the second example, the stopper including the base portion having a semi-cylindrical shape attached to the outer peripheral surface of the housing 30 is exemplified as an inhibition portion that inhibits the displacement or the pressing operation of the operation portion, but the inhibition portion may be a movable or removable cap that covers the entire operation portion. The inhibition portion may be a complete cylindrical body as long as it covers the operation portion. Alternatively, it may be a movable or removable cap that covers a part (for example, the pressing protrusion 37 of the flexible piece 36) of the operation portion. Further, it may be a movable or removable cap that covers the locking part.

Further, the operation portion may extend from the vicinity of the wing 13 toward the base end, or as disclosed in JP 2011-200599 A, the operation portion and the locked part may be provided on the upper surface of the housing instead of the left and right. In this case, a protruding wall may be arranged on the upper surface of the housing located between the locked part and the wing. The direction in which the operation portion extends is not particularly limited, and the number and shape of operation portions are not limited to those in the above-described embodiment.

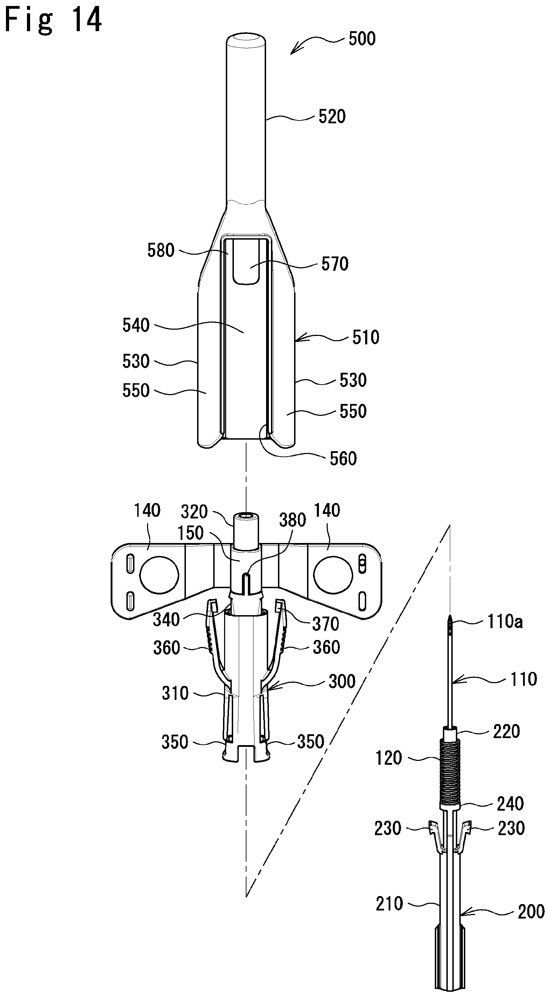

As illustrated in WO 2017/033449 (Fig. 12), the housing may include an operation portion in which the locking part is locked at a first position of the needle hub where the needle protrudes from the cylinder tip, and the locking part is detached when a pressing operation is performed. That is, the operation portion also functions as a locked part. Also in this case, the above-described erroneous operation-inhibiting part can be applied, which can effectively suppress unintended pressing of the operation portion. Further, as illustrated in JP 2007-521080 T (Fig. 14), the housing may include a locked part to which the locking part is locked at the first position of the needle hub and need not include the operation portion. Also in this case, the above-described erroneous operation-inhibiting part can be applied, which can effectively suppress unintended pressing of the locking part.

### [Winged Needle Assembly Set]

### <First Example>

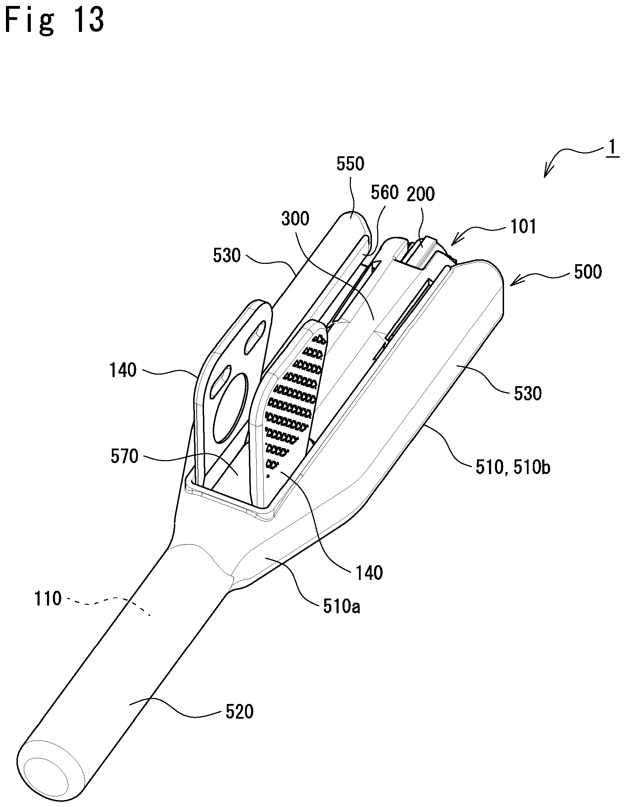

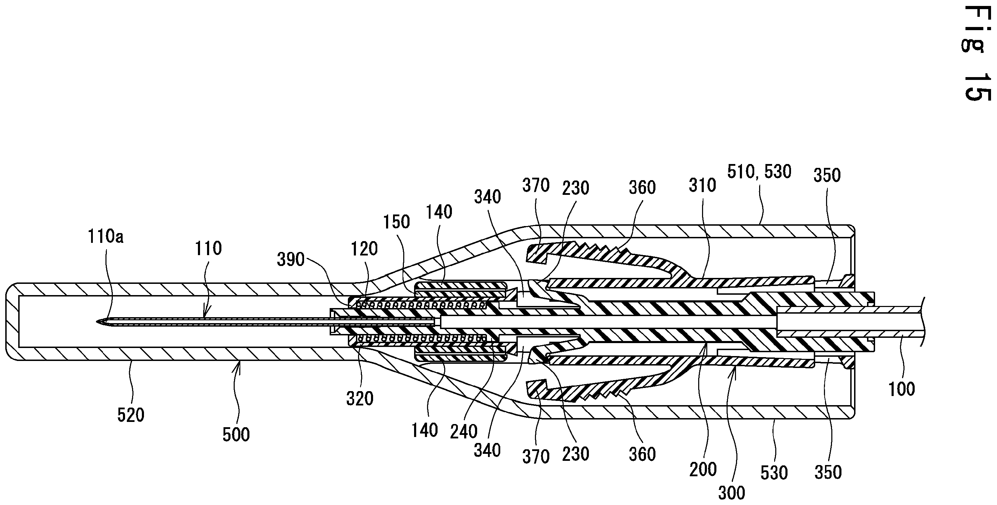

A winged needle assembly set 1 according to a first example will be described in detail with reference to Figs. 13 to 18. Fig. 13 is a perspective view of the winged needle assembly set 1, Fig. 14 is an exploded perspective view of the winged needle assembly set 1, and Fig. 15 is a cross-sectional view of the winged needle assembly set 1.

As illustrated in Figs. 13 to 15, the winged needle assembly set 1 includes a winged needle assembly 101 and a case 500 attached to the winged needle assembly 101. The winged needle assembly 101 includes a needle 110 having a blade face at a tip end, a body having a cylindrical shape to which the needle 110 is fixed, and a pair of wing portions 140 provided in the body. In the present example, the body of the winged needle assembly 101 includes a needle hub 200 to which the base end portion of the needle 110 is fixed, and a housing 300 with a cylindrical shape that supports the needle hub 200. The pair of wing portions 140 are formed with a separate member from the housing 300, and are attached to a tip end portion of the housing 300.

In the present example, a tube 100 (see Fig. 15) is connected to the base end portion of the needle hub 200. In the present description, the tip end side of the winged needle assembly 101 means a needle tip 110a side of the needle 110, and the base end side of the winged needle assembly 101 means the side opposite to the needle tip 110a and to which a pipe such as the tube 100 is connected. Hereinafter, for convenience of explanation, a direction in which the wing portions 140 extend is referred to as a "left-right direction", and a direction orthogonal to the left-right direction and the length direction (axial direction) of the needle 110 is referred to as a "vertical direction". In addition, terms indicating directions such as up and down, left and right, and the like are similarly used for the case 500.

The case 500 is a cap used for the winged needle assembly 101 and is removed when puncturing is performed. In other words, the case 500 is a type of packaging case covering the winged needle assembly 101, and is attached to the winged needle assembly 101 in a storage state until puncture is performed. The case 500 is formed in a cylindrical shape capable of accommodating the winged needle assembly 101 as a whole, and is mounted so as to cover most of the winged needle assembly 101 from the needle tip 110a to the base end of the housing 300. On the other hand, the pair of wing portions 140 extend to the outside of the case 500, and most of the wing portions except the vicinity of the roots are not covered by the case 500. As will be described in detail later, the case 500 is configured to abut on the pair of wing portions 140 to raise the pair of wing portions 140 in the same direction.

The winged needle assembly set 1 has a structure in which the winged needle assembly 101 is inserted into a cylinder of the case 500, and the pair of wing portions 140 largely protrude to the outside of the case 500. The pair of wing portions 140 are raised substantially parallel to each other and upward from the upper end of the case 500. The base end portion of the case 500 is open, and the winged needle assembly 101 is inserted from this opening portion. At the time of performing the puncture operation, the pair of wing portions 140 are bent upward and gripped, but with the winged needle assembly set 1, this state is already achieved by the case 500, so that the pair of wing portions 140 can be stably and quickly gripped at the time of performing the puncture operation.

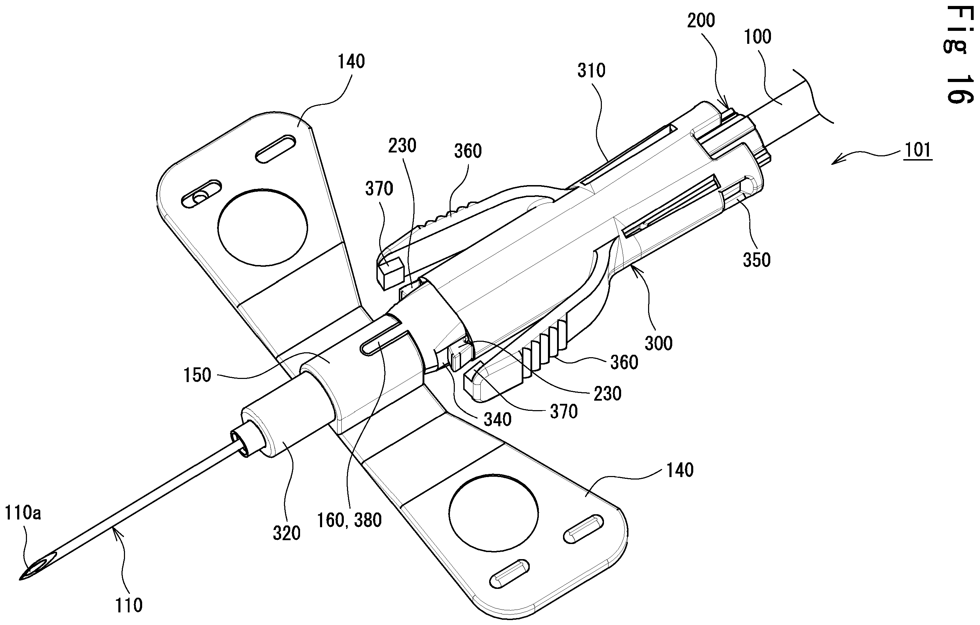

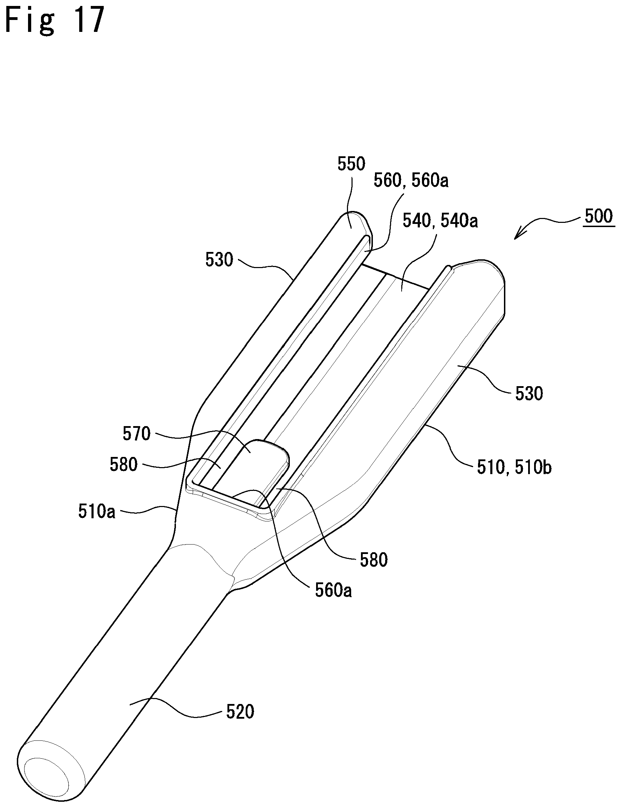

Hereinafter, the winged needle assembly 101 and the case 500 constituting the winged needle assembly set 1 will be described in detail with reference to Figs. 16 and 17 as appropriate.

### [Winged Needle Assembly]

Fig. 16 is a perspective view of the winged needle assembly 101. As illustrated in Figs. 13 to 16, the winged needle assembly 101 includes a needle 110, a needle hub 200 to which a base end portion of the needle 110 is fixed, and a housing 300 having a cylindrical shape into which the needle hub 200 is inserted. The housing 300 supports the needle hub 200 in a slidable state from a first position where the needle 110 protrudes from the cylinder tip, to a second position where the needle 110 is accommodated in the cylinder. The winged needle assembly 101 has the same structure as the winged needle assembly 10 except that the protruding wall 40, which is an erroneous operation-inhibiting part, is not provided. Therefore, the configuration of the winged needle assembly 101 will be briefly described below.

The winged needle assembly 101 further includes an erroneous puncture-preventing mechanism including a spring 120 that is located on the tip end side of the needle hub 200 and that, in a compressed state, generates a biasing force to move the needle hub 200 toward the base end of the housing 300, and an operation portion that releases the compression of the spring 120, and accommodates the needle 110 in the housing 300 using the spring 120 and the operation portion. The housing 300 accommodates the needle 110 after the compression of the spring 120 is released. In the present example, a locking part 230 is formed in the needle hub 200, and the housing 300 is provided with a first opening 340 in which a locking part 230 is locked at the first position of the needle hub 200 and a second opening 350 in which the locking part 230 is locked at the second position of the needle hub 200. In addition, the housing 300 includes a flexible piece 360 as the above operation portion.

In the needle hub 200, a large-diameter portion 210 having a large outer diameter is formed on the base end side with respect to the axial central portion, and a small-diameter portion 220 having a small outer diameter is formed on the tip end side. The pair of engagement portions 230 extend from the boundary between the large-diameter portion 210 and the small-diameter portion 220, and the flange portion 240 is formed on the tip end side of the locking parts 230. In the present example, the spring 120 is externally fitted from the tip end of the needle hub 200 to the small-diameter portion 220, and is arranged in the housing 300 in a state of being compressed by the flange portion 240 and the annular portion 390 (see Fig. 15) formed at the tip end portion of the housing 300.

The housing 300 is made of, for example, a hard resin, and has a base end portion with a substantially elliptical cross section having a long diameter along the left-right direction, a tip end portion having a circular cross section, a base end side having a large-diameter portion 310 having a large outer diameter, and a tip end side having a small-diameter portion 32 having a small outer diameter. A pair of first openings 340, a pair of second openings 350, and a pair of flexible pieces 360 are formed on both left and right sides of the large-diameter portion 310. Pressing protrusions 370 protruding in the direction toward the first opening 340 are formed at the tip end portion of each flexible piece 360.

As described above, the housing 300 is provided with the pair of wing portions 140. The wing portions 140 are used when the winged needle assembly 101 is fixed to an arm or the like of a patient using tape after puncturing with the needle 110, and are also used when puncturing is performed. The wing portions 140 are, for example, formed of a flexible resin, and are attached to the tip end portion of the housing 300. Then, the pair of wing portions 140 greatly extend from the tip end portion of the housing 300 to the left and right beyond the left and right ends of the flexible piece 360. The wing portions 140 have a shape in which the tip end is formed to be wider than the root, but the shape of the wing portions 140 is not particularly limited.

A cylindrical portion 150 is provided at the root of the pair of wing portions 140, and the pair of wing portions 140 are attached to the housing 300 by fitting the cylindrical portion 150 to the outside of the small-diameter portion 320 of the housing 300. That is, the pair of wing portions 140 are connected via the cylindrical portion 150 and attached to the small-diameter portion 320. A projecting strip portion 380 extending toward the tip end is formed at an upper end portion of the small-diameter portion 320, and a recess portion 160 into which the projecting strip portion 380 is fitted is formed at an upper end portion of the cylindrical portion 150. With this uneven structure, the wing portions 140 are positioned in the circumferential direction with respect to the housing 300.

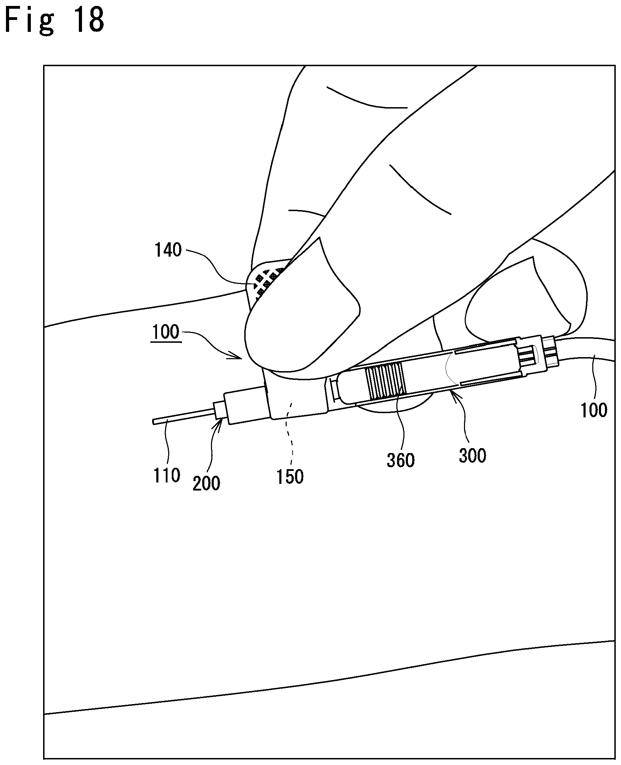

The pair of wing portions 140 are connected to the lower end portion of the cylindrical portion 150 and extend from the lower portion of the small-diameter portion 320 to the left and right. Note that since the pair of wing portions 140 have flexibility, the root portions can be bent such that the tip ends face upward, and as illustrated in Fig. 18 to be described later, the pair of wing portions 140 bent upward are overlapped and gripped when performing puncture. In addition, since the wing portions 140 are elastically deformed, they return to their original shape widened to the left and right when released by the operator's hand.

### [Case]

Fig. 17 is a perspective view of the case 500. As illustrated in Figs. 13 to 15 and Fig. 17, the case 500 has a first cylindrical portion 510 as a base portion that abuts on the pair of wing portions 140 and raises the pair of wing portions 140 in the same direction. In addition, the case 500 has a second cylindrical portion 520 extending from the tip end of the first cylindrical portion 510. The second cylindrical portion 520 is a cap portion that accommodates the needle 110. The case 500 is, for example, an integrally molded product made of a hard resin, and the internal space of the first cylindrical portion 510 communicates with that of the second cylindrical portion 520.

The case 500 has a shape corresponding to the shape of the winged needle assembly 101, and the first cylindrical portion 510 is formed to be larger on the left and right than the second cylindrical portion 520. The second cylindrical portion 520 is formed in a cylindrical shape having a perfectly circular cross section orthogonal to the length direction, and has a substantially constant diameter. On the other hand, the first cylindrical portion 510 is formed in a rectangular cylindrical shape that is longer in the left-right direction than in the vertical direction, and the length in the left-right direction (hereinafter, may be referred to as "width") is shorter on the tip end side close to the second cylindrical portion 520. Note that the vertical length of the first cylindrical portion 510 is equal to or slightly longer than the vertical length of the second cylindrical portion 520.

The second cylindrical portion 520 has a bottomed cylindrical shape with a closed tip end, and accommodates and protects the entire needle 110. As illustrated in Fig. 15, the inner diameter of the second cylindrical portion 520 may be larger than the outer diameter of the small-diameter portion 320 of the housing 300, and the tip end portion of the small-diameter portion 320 may be inserted into the second cylindrical portion 520. In the present example, the small-diameter portion 320 is inserted into the second cylindrical portion 520 with the outer peripheral surface of the small-diameter portion 320 in contact with the inner peripheral surface of the second cylindrical portion 520. This restricts the movement of the winged needle assembly 101 in the vertical and left-right directions in the case 500, which stably attaches the case 500 to the winged needle assembly 101.

The engagement portion between the case 500 and the winged needle assembly 101 is not limited to the small-diameter portion 320, and may be formed at another portion. For example, the tip end of the needle hub and the case 500 may be engaged. Furthermore, the winged needle assembly may be engaged with a peripheral portion of a lower cover 540 or an upper opening 560 described later. Note that it is preferable that the engagement portion between the case 500 and the winged needle assembly 101 is formed on the tip end side instead of the wing portions because the case can be stably removed when it is removed.

The case 500 accommodates, in a cylinder, most of the winged needle assembly 101 from the needle tip 110a to the base end of the housing 300, excluding the pair of wing portions 140. The length of the case 500 is, for example, equal to or slightly longer than that of the winged needle assembly 101. The second cylindrical portion 520 is formed with a length that prevents the needle tip 110a from contacting the bottom inner surface, with the tip end portion of the housing 300 inserted into the cylinder. Note that the tip end of the second cylindrical portion 520 may be open as long as the needle tip 110a does not protrude from the second cylindrical portion 520.

The length of the case 500 may be shorter than that of the winged needle assembly 101, and most of the base end side of the housing 300 may protrude from the case 500, but it is preferable that the operation portion such as the pair of flexible pieces 360 is covered with the case 500. Since the winged needle assembly 101 has an erroneous puncture prevention function, covering the flexible piece 360 with the case 500 makes it possible to effectively prevent the erroneous puncture-preventing mechanism from erroneously operating owing to the flexible piece 360 being pressed erroneously at the time of puncture operation or the like.

The first cylindrical portion 510 is a portion that raises the pair of wing portions 140, accommodates most of the housing 300 including the root portions of the pair of wing portions 140 and the flexible piece 360, and protrudes to the left and right beyond the left and right ends of the second cylindrical portion 520. As described above, the width of the first cylindrical portion 510 is smaller on the tip end side than on the base end side, and a reduced width portion 510a is formed on the tip end side of the first cylindrical portion 510. In the present example, the width of the reduced width portion 510a gradually decreases toward the tip end of the first cylindrical portion 510, but a step that steeply changes the width may be formed in the first cylindrical portion 510.

In the first cylindrical portion 510, a wide portion 510b having a width larger than that of the reduced width portion 510a is formed from a portion corresponding to the tip end portion of the flexible piece 360 to the base end. As illustrated in Fig. 15, the boundary between the reduced width portion 510a and the wide portion 510b is present at a position overlapping each tip end portion of the pair of flexible pieces 360 in the left-right direction. In the wide portion 510b, a distance (W1) between inner surfaces facing each other in the left-right direction is larger than a length (W2) along the left-right direction from a left end of the left flexible piece 360 to a right end of the right flexible piece 360, and the first cylindrical portion 510 is formed so as not to contact with the flexible piece 360.

The first cylindrical portion 510 includes a pair of side wall portions 530 that cover the sides of the housing 300, a lower cover 540 that covers the lower side of the housing 300, and an upper cover 550 that covers the upper side of the housing 300. The pair of side wall portions 530 are formed obliquely with respect to the axial direction of the first cylindrical portion 510 in the reduced width portion 510a, and are formed in parallel with the axial direction of the first cylindrical portion 510 in the wide portion 510b. The pair of side wall portions 530 are arranged to face each other at a distance (W1) larger than the length (W2) described above from the base end of the first cylindrical portion 510 to a position corresponding to the tip end portion of the flexible piece 360.

The lower cover 540 is formed along the left-right direction so as to connect the lower ends of the pair of side wall portions 530 to each other, and widely covers the lower surface of the housing 300. Providing the lower cover 540 protects the lower portion of the housing 300 and also connects the pair of side wall portions 530, which improves the mechanical strength of the case 500. Further, the lower cover 540 may be provided with a recess portion 540a into which the large-diameter portion 310 of the housing 300 is fitted along the axial direction of the first cylindrical portion 510. The recess portion 540a functions as a guide when, for example, the case 500 is attached to the winged needle assembly 101.

The upper cover 550 extends inward in the left-right direction from the upper ends of the pair of side wall portions 530 and is formed substantially parallel to the lower cover 540. The upper cover 550 covers the upper side of the flexible piece 360 of the housing 300 and does not substantially cover the upper surface of the large-diameter portion 310. That is, the upper surface of the large-diameter portion 310 is not covered with the case 500 and is largely exposed. The first cylindrical portion 510 is provided with an upper opening 560 for exposing the upper surface of the large-diameter portion 310. By forming the upper opening 560, the case 500 can be attached to the winged needle assembly 101 with the pair of wing portions 140 raised.

The upper opening 560 is formed in a substantially rectangular shape in plan view from the base end of the first cylindrical portion 510 to the vicinity of the second cylindrical portion 520 along the axial direction of the case 500. In addition, the upper opening 560 is formed to have, for example, a width slightly larger than the width of the cylindrical portion 150 to which the pair of wing portions 140 are connected. Since the pair of wing portions 140 are bent at the roots (connection portion with the cylindrical portion 150), in this case, the pair of wing portions 140 that are bent and raised upward easily extend straight from the upper opening 560. On both left and right sides of the upper opening 560, a pair of upper covers 550 are formed with the same width.

In the present example, the pair of raised wing portions 140 abut on a tip end edge 560a of the upper opening 560. As a result, the movement of the winged needle assembly 101 toward the tip end of the case 500 is restricted. The upper opening 560 is formed to have a length that, for example, allows the tip end portion of the housing 300 to be inserted into the second cylindrical portion 520 when the wing portions 140 abut on the tip end edge 560a. A side edge 560b of the upper opening 560 (inner end edge of the upper cover 550) is formed linearly along the axial direction of the case 500 and abuts on surfaces facing outward in the left-right direction of the pair of raised wing portions 140. That is, the pair of raised wing portions 140 are supported from the outside by the side edge 560b, and the raised state of the wing portions 140 is maintained.

The first cylindrical portion 510 is provided with a tongue piece portion 570 extending from the tip end edge 560a of the upper opening 560 toward the base end. The tongue piece portion 570 may extend so as to cover the upper side of the cylindrical portion 150 and may be in contact with the upper surface of the cylindrical portion 150. In this case, the cylindrical portion 150 of the housing 300 is pressed from above by the tongue piece portion 570, and for example, the case 500 is firmly and stably mounted. The vertical interval between the tongue piece portion 570 and the lower cover 540 is equal to or slightly smaller than the vertical length of the cylindrical portion 150. In addition, between the tongue piece portion 570 and the side edge 560b, a groove 580 through which the raised wing portions 140 can be inserted is formed. The groove 580 is formed on both left and right sides of the tongue piece portion 570, and the pair of wing portions 140 are arranged so as to sandwich the tongue piece portion 570. In the winged needle assembly set 1, the pair of wing portions 140 are raised upward with the vicinity of the roots sandwiched between the side edge 560b and the tongue piece portion 570.

As described above, the first cylindrical portion 510 includes the upper opening 560 that is an opening portion for extending the pair of wing portions 140 to the outside of the case 150, and the tongue piece portion 570 that extends toward the base end from the tip end edge 560a, which is an edge of the upper opening 560 and presses the cylindrical portion 150 of the winged needle assembly 101. The first cylindrical portion 510 is formed between the side edge 560b of the upper opening 560 and the tongue piece portion 570, and has the groove 580 through which the pair of wing portions 140 are inserted. The tongue piece portion 570 is formed to have, for example, a length sufficient to cover the cylindrical portion 150. Lengthening the tongue piece portion 570 makes it easy to prevent the case 500 from coming off, but lengthening the tongue piece portion too much may cause a defect such as damage due to contact of the tip end of the needle 110 with the tongue piece portion 570 when the winged needle assembly 101 is pulled out from the case 500. Further, in order to prevent the deflection of the upper opening 560, the edge of the upper opening 560 may have a thick shape, and walls along the vertical direction may be formed in the tip end edge 560a and the side edge 560b.

Fig. 18 is a view illustrating a state of puncture operation using the winged needle assembly 101. As illustrated in Fig. 18, when a blood vessel of a patient is punctured with the needle 110, the pair of wing portions 140 are gripped with them folded upward and overlapped. That is, the pair of wing portions 140 are gripped with them bent upward so as to sandwich the cylindrical portion 150. With the winged needle assembly set 1 having the above-described configuration, since the pair of wing portions 140 extend out of the case 500 in a state of being raised upward, a medical worker can stably and quickly grip the wing portions 140. That is, a state in which the wing portions 140 are raised in the same direction is realized by the case 500.

In addition, in the case 500, since the first cylindrical portion 510 that raises the wing portions 140 and the second cylindrical portion 520 that accommodates the needle 110 are integrated, the case 500 can be removed by pulling the second cylindrical portion 520 toward the tip end after the pair of wing portions 140 are stably gripped. In particular, since the case 500 covers substantially the entire winged needle assembly 101 except for the wing portions 140, there is nothing to grip other than the wing portions 140, which allows this procedure to be performed more reliably.

In addition, in the winged needle assembly set 1, since the pair of flexible pieces 360 are covered by the case 500 in addition to the pair of wing portions 140 raised upward, the wing portions 140 are gripped in a state in which the flexible pieces 360 are unlikely to be erroneously pressed. When the wing portions 140 are gripped so as to be bent, the wing portions 140 or a finger may contact with the flexible pieces 360 to erroneously press the flexible pieces 360 and erroneously operate the erroneous puncture-preventing mechanism, but in the case of the winged needle assembly set 1, such erroneous operation is effectively suppressed. Note that the case 500 prevents the flexible piece 360 from being pressed during conveyance or storage.

### <Second Example>

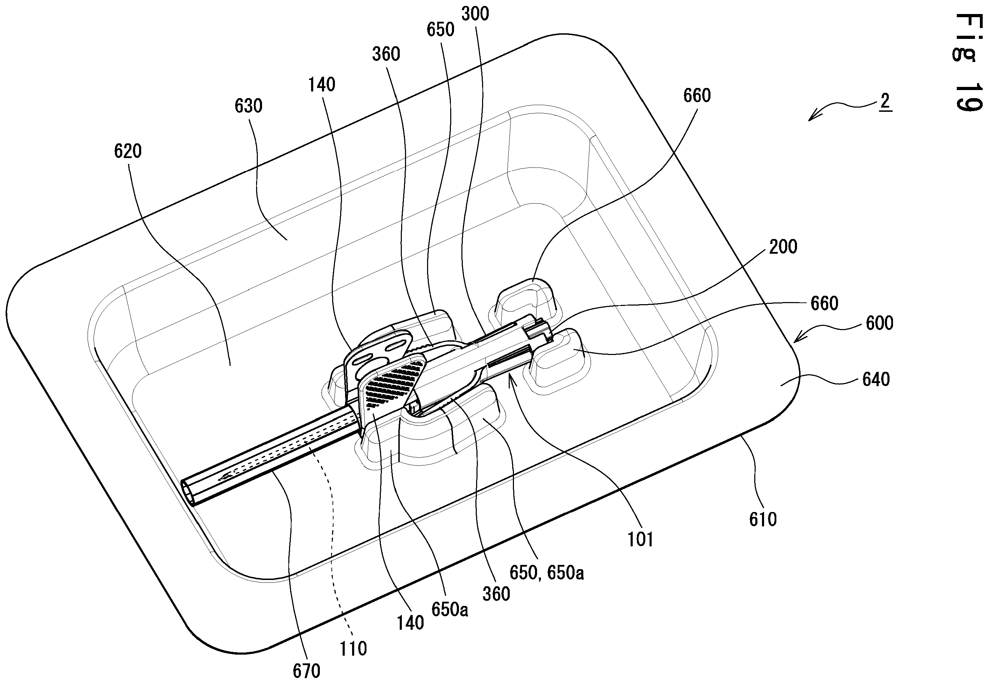

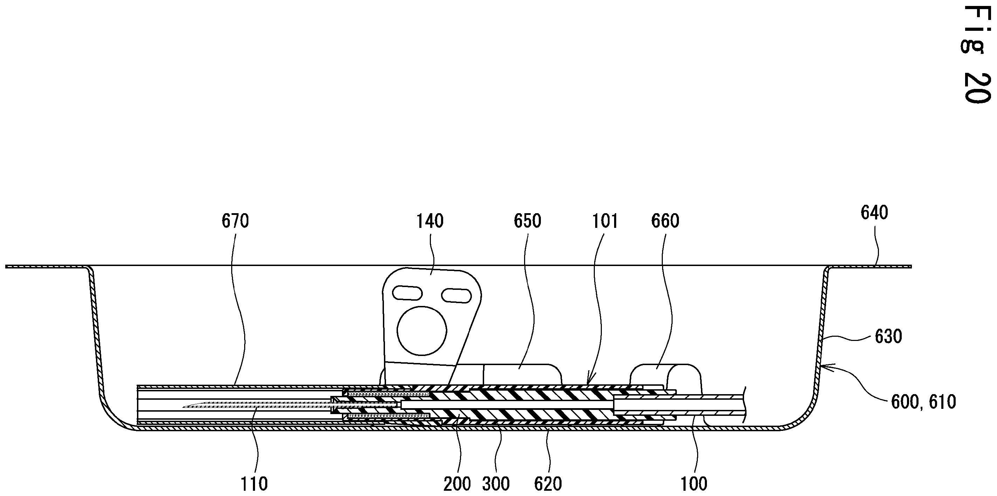

A winged needle assembly set 2 according to a second example will be described in detail with reference to Figs. 19 and 20. Fig. 19 is a perspective view of the winged needle assembly set 2, and Fig. 20 is a cross-sectional view of the winged needle assembly set 2. Hereinafter, the same reference numerals are used for the same or similar components as those in the first example, and redundant description will be omitted.

As illustrated in Figs. 19 and 20, the winged needle assembly set 2 includes a case 600 as a packaging case covering the winged needle assembly 101. The case 600 includes a case body 610 that accommodates the winged needle assembly 101, and a pair of first holding portions 650 that stand on a bottom portion 620 of the case body 610, abut on the pair of wing portions 140 to raise the pair of wing portions 140 in the same direction, and sandwich the winged needle assembly 101. In addition, the case 600 may be provided with a pair of second holding portions 660 that sandwich the base end portion of the housing 300. Similarly to the first holding portions 650, the second holding portions 660 stand on the bottom portion 620 of the case body 610. In the present example, since a cap 670 to be described later is used as a cap for accommodating the needle 110, the case 600 does not have a configuration like the tongue piece portion 570 described above.

The case body 610 has a container shape with an open upper portion and accommodates the entire winged needle assembly 101. The case body 610 includes a bottom portion 620 having a substantially rectangular shape in plan view, side portions 630 standing on a peripheral portion of the bottom portion 620, and a flange portion 640 protruding outward in parallel with the bottom portion 620 from upper ends of the side portions 630. The bottom portion 620 is formed flat except for a portion where the holding portion is formed, and is formed having sufficient size to accomodate the winged needle assembly 101.

The side portions 630 are formed in a rectangular cylindrical shape and surround four sides of the winged needle assembly 101. In addition, the side portions 630 are formed at a height at which the pair of raised wing portions 140 do not protrude from the opening portion of the case body 610. That is, the bottom portion 620 and the side portions 630 form a space separated from the periphery that can accommodate the entire winged needle assembly 101. For example, a sheet for sealing the opening portion of the case body 610 may be attached to the flange portion 640, and the internal space of the case 600 accommodating the winged needle assembly 101 may be sealed.

The pair of first holding portions 650 are arranged facing the central portion of the bottom portion 620 at a distance where the housing 300 of the winged needle assembly 101 can be sandwiched. The pair of first holding portions 650 are walls standing on the bottom portion 620, and include wall portions 650a that sandwich a portion of the housing 300 to which the wing portions 140 are attached from the left and right with the wing portions 140 bent and raised at the roots, and wall portions 650b that extend from the wall portions 650a toward the second holding portions 660 and cover the flexible piece 360. The pair of wall portions 650a are formed in parallel to each other, while the pair of wall portions 650b have a shape bulging in directions away from each other. Although the side portions 630 and the first holding portions 650 are formed apart from each other, the side portions 630 may be formed as one wall so as to play a role of the first holding portions 650. In this case, the case can be downsized.

The wall portions 650a are in contact with surfaces of the pair of raised wing portions 140 facing outward in the left-right direction. That is, the pair of raised wing portions 140 are supported from the outside by the wall portions 650a, and the raised state of the wing portions 140 is maintained. The interval between the pair of wall portions 650a is set to be equal to or slightly larger than, for example, the length obtained by adding the outer diameter of the cylindrical portion 150 to the thickness of the root portions of the pair of wing portions 140. The pair of wall portions 650b are formed at intervals allowing insertion of portions of the housing 300 where the flexible pieces 360 are formed while securing a gap with the flexible pieces 360 to such an extent that the flexible pieces 360 do not contact. Note that the wall portions 650b are curved along the flexible pieces 360.

The pair of first holding portions 650 are formed at a height exceeding the upper surface of the winged needle assembly 101. On the other hand, the first holding portions 650 are preferably formed at a height not exceeding the central portion in the length direction of the wing portions 140 in the raised state so as to allow easy gripping of the wing portions 140. In the present example, the pair of wall portions 650a, the pair of wall portions 650b, and the second holding portions 660 are all formed at the same height, but the heights of the walls may be different from each other. The pair of second holding portions 660 are arranged to face each other with a space therebetween into which the base end portion of the housing 300 can be inserted so as to press two corners of the base end portion of the housing 300. In the case 600, the winged needle assembly 101 is stably held by the two holding portions.

In the present example, the cap 670 for accommodating the needle 110 is attached to the tip end portion of the housing 300. The cap 670 is configured as a separate member from the case 600, and is removed after taking out the winged needle assembly 101 from the case 600 with the wing portions 140 gripped. In a state in which the winged needle assembly 101 is accommodated in the case 600, the cap 670 cannot be removed because the tip end of the cap 670 and the side portions 630 of the case body 610 are close to each other.

With the winged needle assembly set 2 having the above-described configuration, similarly to the winged needle assembly set 1, since the pair of wing portions 140 are in a state of being raised upward, the wing portions 140 can be stably and quickly gripped at the time of puncture operation. In addition, since the cap 670 cannot be removed with the winged needle assembly 101 accommodated in the case 600, the operation of removing the cap 670 can be executed more reliably with the pair of wing portions 140 stably gripped. In addition, since the pair of flexible pieces 360 are covered with the first holding portions 650, the wing portions 140 are gripped in a state in which the flexible piece 360 is unlikely to be erroneously pressed, which effectively suppresses the erroneous operation of the erroneous puncture-preventing mechanism at the time of the puncture operation.

Note that the above-described examples can be appropriately modified in design without impairing the object of the present invention. For example, although the packaging case of the above example covers most of the winged needle assembly 101, the packaging case may be attached only to the root portions of the wing portions 140. In addition, the holding portions that hold the wing portions in the raised state may extend from the side portions, instead of extending from the bottom portion. In addition, as a separate body from the packaging case, a clip-like member that sandwiches the root portions of the pair of wing portions 140 from the left and right may be provided, which causes the wing portions to be in a raised state. In this case as well, it is preferable that the wing portions are in a raised state while being accommodated in the packaging case. In addition, the winged needle assembly according to the present invention may include a detachable holding portion that holds the pair of wing portions in a state of being raised in the same direction. The holding portion is removed after the wing portions are gripped at the time of the puncture operation or after the puncture operation is performed.

The winged needle assembly to which the present invention is applied is not limited to the winged needle assembly 101. The winged needle assembly may include, for example, an operation portion extending from the vicinity of the wing portions toward the base end, and may include a housing in which the operation portion and the locked part are provided on the upper surface instead of the left and right, as illustrated in JP 2011-2000599 A.

As illustrated in WO 2017/033449 (Fig. 12), the housing of the winged needle assembly may include an operation portion in which the locking part is locked at a first position of the needle hub where the needle protrudes from the cylinder tip, and the locking part is detached when a pressing operation is performed. That is, the operation portion also functions as a locked part. In this case, it is preferable that the packaging case covers the operation portion of the housing as in the above-described example. As illustrated in JP 2007-521080 T (Fig. 14), the housing may include a locked part to which the locking part is locked at the first position of the needle hub and may not include the operation portion. In this case, the packaging case preferably covers the locked part of the housing.

In addition, the winged needle assembly to which the present invention is applied may be an assembly that does not include a spring-type erroneous puncture-preventing mechanism. For example, as disclosed in JP 2020-62507 A, it may be a winged needle assembly including a non-spring type erroneous puncture-preventing mechanism in which a needle tip is accommodated in a cylinder of a housing by pinching a stopper provided on a needle hub and sliding the needle hub toward a base end. The winged needle assembly may not include the erroneous puncture-preventing mechanism and the needle may not move. In addition, the present invention can also be applied to an indwelling needle, such as a double needle type including an inner needle and an outer needle or an L-shaped needle, in which a needle extends in the same direction as raised wing portions.

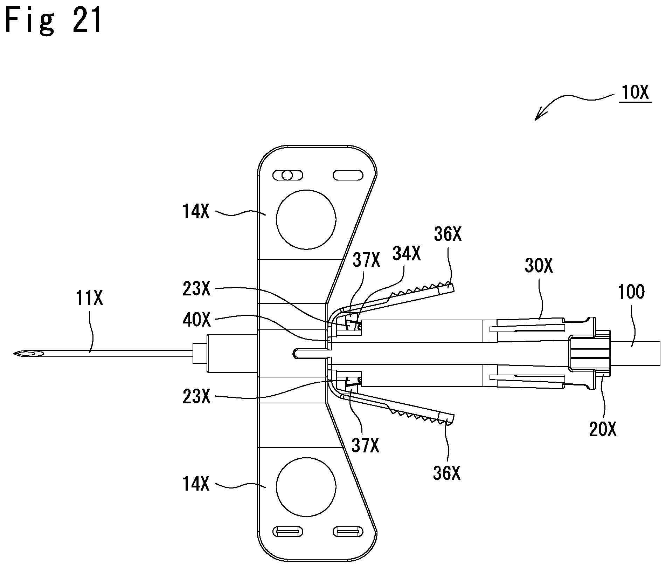

The winged needle assembly according to one aspect of the present invention may be a winged needle assembly 10X illustrated in Fig. 21. Similarly to the above-described winged needle assemblies, the winged needle assembly 10X includes a needle 11X, a needle hub 20X to which a base end portion of the needle 11X is fixed, a housing 30X having a cylindrical shape into which the needle hub 20X is inserted, and a pair of wing portions 14X. The needle hub 20X is provided with locking parts 23X, and the housing 30X is provided with first openings 34X to which the locking parts 23X are locked at a first position of the needle hub 20X. In addition, the housing 30X includes flexible pieces 36X as operation portions that press the locking parts 23X. The flexible pieces 36X are arranged to face the locking parts 23X, press the locking parts 36X from the outside of the housing 30X, and move the locking parts 36X into the cylinder of the housing 30X.

The flexible pieces 36X are similar to the flexible pieces of the above-described winged needle assemblies in that they are formed in an arm shape extending from the outer peripheral surface of the housing 30X and are provided on each of the left and right sides of the housing 30X. On the other hand, the flexible pieces 36X are different from the flexible pieces of the above-described winged needle assemblies in that they are formed to extend in the direction toward the base end from the wing portions 14X (wing) side of the housing 30X and so that the distance between the flexible pieces 36X and the outer peripheral surface of the housing 30X gradually increase toward the base end. The flexible pieces 36X each have a free end (tip end) located on the base end side of the assembly away from the wing and the locking parts 36X. The root portions of the flexible pieces 36X are preferably provided at a position adjacent to the wing portions 14X on the outer peripheral surface of the housing 30X. In addition, the root portions of the pair of flexible pieces 36X are connected to each other by a wall portion 40X extending in the left-right direction. Pressing protrusions 37X that press the locking parts 23X inward are formed at positions facing the locking parts 23X in the vicinity of the root of each flexible piece 36X.

Also in the winged needle assembly 10X, the free end side (tip end portion) of the flexible piece 36X is pressed when the needle 11X is accommodated in the housing 30X, but in the winged needle assembly 10X, since the free end of the flexible piece 36X is separated from the wing portions 14, unintended pressing of the flexible piece 36X is unlikely to occur at the time of puncture operation. In addition, the wall portion 40X functions as an erroneous operation-inhibiting part that suppresses an erroneous operation of the erroneous puncture-preventing mechanism due to mishandling of the flexible piece 36X. The wall portion 40X is a wall positioned between the wing portions 14X and the first openings 34X, and protrudes in the left-right direction intersecting the axial direction of the housing 30. The wall portion 40X preferably protrudes to the left and right beyond the tip ends of the locking parts 23X fitted to the first openings 34X.

### REFERENCE SIGNS LIST

- 10, 50, 55, 60, 65: Winged Needle Assembly
- 11: Needle
- 11a: Needle Tip
- 12: Spring
- 13: Wing
- 14: Wing Portion
- 15: Cylindrical Portion
- 16, 69: Recess Portion
- 20: Needle Hub
- 21, 31: Large-Diameter Portion
- 22, 32: Small-Diameter Portion
- 23: Locking Part
- 24: Flange Portion
- 25, 39: Projecting Strip Portion
- 26: Step
- 30: Housing
- 33a, 33b: Opening
- 34: First Opening
- 35: Second Opening
- 36: Flexible Piece
- 37: Pressing Protrusion
- 38: Inclined Wall Portion
- 40: Protruding Wall
- 41: Annular Portion
- 51: Erroneous Operation Suppressing Member
- 56: Erroneous Operation Suppression Cylindrical Portion
- 61, 66: Stopper
- 62, 67: Base Portion
- 63: Protrusion
- 64, 68: Gripping Portion
- 100: Tube
- 1, 2: Winged Needle Assembly Set
- 101: Winged Needle Assembly
- 110: Needle
- 110a: Needle Tip
- 120: Spring
- 140: Wing Portion
- 150: Cylindrical Portion
- 160, 540a: Recess Portion
- 200: Needle Hub
- 210, 310: Large-Diameter Portion
- 220, 320: Small-Diameter Portion
- 230: Locking Part
- 240: Flange Portion
- 300: Housing
- 340: First Opening
- 350: Second Opening
- 360: Flexible Piece
- 370: Pressing Protrusion
- 380: Projecting Strip Portion
- 390: Annular Portion
- 500,600: Case
- 510: First Cylindrical Portion
- 510a: Reduced Width Portion
- 510b: Wide Portion
- 520: Second Cylindrical Portion
- 530: Side Wall Portion
- 540: Lower Cover
- 550: Upper Cover
- 560: Upper Opening
- 560a: Tip End Edge
- 560b: Side Edge
- 570: Tongue Piece Portion
- 580: Groove
- 610: Case Body
- 620: Bottom Portion
- 630: Side Portion
- 640: Flange Portion
- 650: First Holding Portion
- 650a, 650b: Wall Portion
- 660: Second Holding Portion
- 670: Cap

## Claims

1. A winged needle assembly (10) provided with an erroneous puncture-preventing mechanism for a needle (11), the winged needle assembly (10) comprising:
the needle (11);
a needle hub (20) to which a base end portion of the needle (11) is fixed and in which a locking part (23) is formed;
a housing (30) with a cylindrical shape that supports the needle hub (20) at a first position where the needle (11) protrudes from a cylinder tip;
a spring (12) that biases the needle hub (20) toward a base end of the housing (30);
a wing (13) provided to the housing (30); and
an erroneous operation-inhibiting part for suppressing an erroneous operation of the erroneous puncture-preventing mechanism, wherein
the housing (30) includes
a locked part (34) to which the locking part (23) is locked at the first position of the needle hub (20), and
an operation portion (36) for pressing the locking part (23) to detach the locking part (23) from the locked part (34), and
the erroneous operation-inhibiting part is a protruding wall (40) located between the wing (13) and the locked part (34), wherein the protruding wall (40) is formed between the locked part (34) and the wing (13) on an outer peripheral surface of the housing (30) and protrudes outward in a direction intersecting an axial direction of the housing (30) beyond a tip end of the locking part (23) when the same is locked to the locked part (34) so as to serve as a barrier to suppress an unintended pressing operation of the operation portion (36).

2. The winged needle assembly (10) according to claim 1, wherein
the wing (13) includes a pair of wing portions (14) extending in a direction orthogonal to the axial direction of the housing (30) and a cylindrical portion (15) to which the pair of wing portions (14) are connected and that are externally fitted to the housing (30), and
the protruding wall (40) is provided in the cylindrical portion (15) of the wing (13) and protrudes to a position overlapping at least the tip end of the locking part (23) in the axial direction of the housing (30).

3. The winged needle assembly (10) according to claim 1 or 2, wherein
the locked part is a first opening (34) into which the locking part (23) of the needle hub (20) is fitted in the first position of the needle hub (20),
the operation portion is formed as a flexible piece (36) that is formed in an arm shape extending from an outer peripheral surface of the housing (30) to a position facing the first opening (34).

4. The winged needle assembly (10) according to claim 3, wherein
a first opening (34) is formed in the housing (30) on each of left and right sides in a radial direction of the housing (30),
a flexible piece (36) is formed on each of the left and right sides of the housing (30) in the radial direction and extends to a position facing the first opening (34),
the pair of flexible pieces (36) are configured to be elastically deformed in the radial direction of the housing (30), and
a pressing protrusion (37) protruding in a direction of the first opening (34) is formed at a tip end portion of each flexible piece (36) such that the pressing protrusions (37) press the locking parts (23) of the needle hub (20) fitted to the first openings (34) when the flexible pieces (36) are pressed.

## Patentansprüche

1. Nadelanordnung mit Flügeln (10), die mit einem Mechanismus zur Verhinderung eines Fehlstichs für eine Nadel (11) versehen ist, wobei die Nadelanordnung mit Flügeln (10) umfasst:
die Nadel (11);
eine Nadelnabe (20), an der ein basisseitiger Endabschnitt der Nadel (11) befestigt ist und in der ein Verriegelungsteil (23) gebildet ist;
ein Gehäuse (30) mit zylindrischer Form, das die Nadelnabe (20) in einer ersten Position trägt, in der die Nadel (11) aus einer Zylinderspitze vorsteht;
eine Feder (12), die die Nadelnabe (20) zu einem basisseitigen Ende des Gehäuses (30) hin vorspannt;
einen Flügel (13), der an dem Gehäuse (30) vorgesehen ist; und
ein Fehlbetätigungshemmteil zum Unterdrücken einer Fehlbetätigung des Mechanismus zur Verhinderung eines Fehlstichs, wobei
das Gehäuse (30) umfasst
ein Gegenverriegelungsteil (34), an dem das Verriegelungsteil (23) in der ersten Position der Nadelnabe (20) verriegelt ist, und
einen Betätigungsabschnitt (36) zum Drücken des Verriegelungsteils (23), um das Verriegelungsteil (23) von dem Gegenverriegelungsteil (34) zu lösen, und
das Fehlbetätigungshemmteil eine vorstehende Wand (40) ist, die sich zwischen dem Flügel (13) und dem Gegenverriegelungsteil (34) befindet, wobei die vorstehende Wand (40) zwischen dem Gegenverriegelungsteil (34) und dem Flügel (13) an einer Außenumfangsfläche des Gehäuses (30) gebildet ist und in einer Richtung, die eine Axialrichtung des Gehäuses (30) schneidet, über ein spitzenseitiges Ende des Verriegelungsteils (23), wenn dieses an dem Gegenverriegelungsteil (34) verriegelt ist, hinaus nach außen vorsteht, um als Barriere zu dienen, um eine unbeabsichtigte Drückbetätigung des Betätigungsabschnitts (36) zu unterdrücken.

2. Nadelanordnung mit Flügeln (10) nach Anspruch 1, wobei
der Flügel (13) ein Paar Flügelabschnitte (14), die sich in einer Richtung orthogonal zu der Axialrichtung des Gehäuses (30) erstrecken, und einen zylindrischen Abschnitt (15) umfasst, mit dem das Paar Flügelabschnitte (14) verbunden ist und der außen auf das Gehäuse (30) aufgesetzt ist, und
die vorstehende Wand (40) in dem zylindrischen Abschnitt (15) des Flügels (13) vorgesehen ist und bis zu einer Position vorsteht, die zumindest das spitzenseitige Ende des Verriegelungsteils (23) in der Axialrichtung des Gehäuses (30) überlappt.

3. Nadelanordnung mit Flügeln (10) nach Anspruch 1 oder 2, wobei
das Gegenverriegelungsteil eine erste Öffnung (34) ist, in die das Verriegelungsteil (23) der Nadelnabe (20) in der ersten Position der Nadelnabe (20) eingepasst ist,
der Betätigungsabschnitt als flexibles Element (36) gebildet ist, das in einer Armform gebildet ist, die sich von einer Außenumfangsfläche des Gehäuses (30) bis zu einer Position erstreckt, die der ersten Öffnung (34) gegenüberliegt.

4. Nadelanordnung mit Flügeln (10) nach Anspruch 3, wobei
eine erste Öffnung (34) in dem Gehäuse (30) jeweils auf der linken und der rechten Seite in einer Radialrichtung des Gehäuses (30) gebildet ist,
ein flexibles Element (36) jeweils auf der linken und der rechten Seite des Gehäuses (30) in der Radialrichtung gebildet ist und sich bis zu einer Position erstreckt, die der ersten Öffnung (34) gegenüberliegt,
das Paar flexibler Elemente (36) ausgebildet ist, um in der Radialrichtung des Gehäuses (30) elastisch verformt zu werden, und
ein Drückvorsprung (37), der in einer Richtung der ersten Öffnung (34) vorsteht, an einem spitzenseitigen Endabschnitt jedes flexiblen Elements (36) gebildet ist, so dass die Drückvorsprünge (37) die in die ersten Öffnungen (34) eingepassten Verriegelungsteile (23) der Nadelnabe (20) drücken, wenn die flexiblen Elemente (36) gedrückt werden.

## Revendications

1. Ensemble aiguille à ailettes (10) muni d'un mécanisme de prévention de piqûre erronée pour une aiguille (11), l'ensemble aiguille à ailettes (10) comprenant :
l'aiguille (11) ;
une embase d'aiguille (20) à laquelle une partie d'extrémité de base de l'aiguille (11) est fixée et dans laquelle une partie de verrouillage (23) est formée ;
un boîtier (30) de forme cylindrique qui supporte l'embase d'aiguille (20) dans une première position où l'aiguille (11) fait saillie d'une pointe de cylindre ;
un ressort (12) qui sollicite l'embase d'aiguille (20) vers une extrémité de base du boîtier (30) ;
une ailette (13) prévue sur le boîtier (30) ; et
une partie d'inhibition d'actionnement erroné destinée à supprimer un actionnement erroné du mécanisme de prévention de piqûre erronée, dans lequel
le boîtier (30) comprend
une partie verrouillée (34) à laquelle la partie de verrouillage (23) est verrouillée dans la première position de l'embase d'aiguille (20), et
une partie d'actionnement (36) destinée à presser la partie de verrouillage (23) pour détacher la partie de verrouillage (23) de la partie verrouillée (34), et
la partie d'inhibition d'actionnement erroné est une paroi saillante (40) située entre l'ailette (13) et la partie verrouillée (34), dans lequel la paroi saillante (40) est formée entre la partie verrouillée (34) et l'ailette (13) sur une surface périphérique externe du boîtier (30) et fait saillie vers l'extérieur dans une direction croisant une direction axiale du boîtier (30) au-delà d'une extrémité de pointe de la partie de verrouillage (23) lorsque celle-ci est verrouillée à la partie verrouillée (34) de manière à servir de barrière pour supprimer une opération de pression involontaire de la partie d'actionnement (36).

2. Ensemble aiguille à ailettes (10) selon la revendication 1, dans lequel
l'ailette (13) comprend une paire de parties d'ailette (14) s'étendant dans une direction orthogonale à la direction axiale du boîtier (30) et une partie cylindrique (15) à laquelle la paire de parties d'ailette (14) est reliée et qui est emmanchée extérieurement sur le boîtier (30), et
la paroi saillante (40) est prévue dans la partie cylindrique (15) de l'ailette (13) et fait saillie jusqu'à une position chevauchant au moins l'extrémité de pointe de la partie de verrouillage (23) dans la direction axiale du boîtier (30).

3. Ensemble aiguille à ailettes (10) selon la revendication 1 ou 2, dans lequel
la partie verrouillée est une première ouverture (34) dans laquelle la partie de verrouillage (23) de l'embase d'aiguille (20) est ajustée dans la première position de l'embase d'aiguille (20),
la partie d'actionnement est formée sous la forme d'une pièce flexible (36) qui est formée en forme de bras s'étendant d'une surface périphérique externe du boîtier (30) jusqu'à une position en regard de la première ouverture (34).

4. Ensemble aiguille à ailettes (10) selon la revendication 3, dans lequel
une première ouverture (34) est formée dans le boîtier (30) de chacun des côtés gauche et droit dans une direction radiale du boîtier (30),
une pièce flexible (36) est formée de chacun des côtés gauche et droit du boîtier (30) dans la direction radiale et s'étend jusqu'à une position en regard de la première ouverture (34),
la paire de pièces flexibles (36) est configurée pour être déformée élastiquement dans la direction radiale du boîtier (30), et
une saillie de pression (37) faisant saillie dans une direction de la première ouverture (34) est formée au niveau d'une partie d'extrémité de pointe de chaque pièce flexible (36) de sorte que les saillies de pression (37) pressent les parties de verrouillage (23) de l'embase d'aiguille (20) ajustées dans les premières ouvertures (34) lorsque les pièces flexibles (36) sont pressées.
